(19) Europäisches Patentamt
European Patent Office
Office européen des brevets

(11) Veröffentlichungsnummer : **0 101 408**
**B1**

(12) **EUROPÄISCHE PATENTSCHRIFT**

(45) Veröffentlichungstag der Patentschrift : **07.05.86**

(51) Int. Cl.[4] : **C 09 B 29/01**, C 07 C147/12// D06P3/24

(21) Anmeldenummer : **83810319.0**

(22) Anmeldetag : **13.07.83**

(54) **Monoazofarbstoffe, deren Herstellung und Verwendung.**

(30) Priorität : **19.07.82 CH 4397/82**

(43) Veröffentlichungstag der Anmeldung : **22.02.84 Patentblatt 84/08**

(45) Bekanntmachung des Hinweises auf die Patenterteilung : **07.05.86 Patentblatt 86/19**

(84) Benannte Vertragsstaaten : **BE CH DE FR GB IT LI**

(56) Entgegenhaltungen :
**FR-A- 2 162 062**
**US-A- 2 449 130**
**US-A- 2 551 887**
**US-A- 3 692 769**
**US-A- 4 052 444**

(73) Patentinhaber : **CIBA-GEIGY AG**
**Klybeckstrasse 141**
**CH-4002 Basel (CH)**

(72) Erfinder : **Hurter, Rudolf**
**Laufenburgerstrasse 10/3**
**CH-4058 Basel (CH)**

**EP 0 101 408 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die der vorliegenden Erfindung zugrunde liegende Aufgabe war es, neue Monoazofarbstoffe zu finden, die zum Färben von natürlichen und synthetischen Polyamiden aus wässrigem Bad geeignet sind, die ferner Nuancen im Bereich Gelb bis Rot haben, und die ausserdem verbesserte Echtheitseigenschaften, insbesondere in der Lichtechtheit, aufweisen.

Es wurde nun gefunden, dass die Monoazofarbstoffe der Formel (I) den genannten Anforderungen genügen.

Gegenstand der vorliegenden Erfindung sind somit Monoazofarbstoffe der Formel

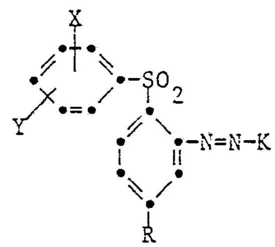

(1),

worin K der Rest einer Kupplungskomponente der Benzol- oder Naphthalinreihe oder der heterocyclischen Reihe und R eine

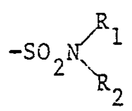 , 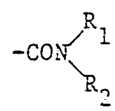 ,

—CO—$R_2$ oder —$SO_2$—O—$R_2$-Gruppe ist, wobei $R_1$ Wasserstoff oder $C_{1-4}$-Alkyl und $R_2$ einen gegebenenfalls substituierten Monosulfo-$C_{1-12}$-Alkylrest, einen Rest der Formel

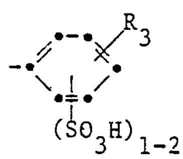

(2)

oder einen Rest der Formel

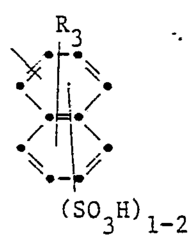

(3),

worin $R_3$ in den Formeln (2) und (3) Wasserstoff, $C_{1-4}$-Alkyl, $C_{1-4}$-Alkoxy, Halogen, $C_{2-6}$-Alkanoylamino oder Carboxy ist, bedeuten und worin X und Y unabhängig voneinander Wasserstoff, $C_{1-4}$-Alkyl, $C_{1-4}$-Alkoxy, Halogen, $C_{2-6}$-Alkanoylamino, $C_{1-5}$-Alkylsulfonylamino oder einen gegebenenfalls substituierten Phenyl- oder Phenoxyrest bedeuten.

Der Rest K einer Kupplungskomponente kann die bei Azofarbstoffen üblichen Substituenten enthalten, z. B. Alkylgruppen mit 1 bis 4 Kohlenstoffatomen, wie Methyl, Aethyl, Propyl, Isopropyl, Butyl, Isobutyl, sek.-Butyl und tert.-Butyl, Alkoxygruppen mit 1 bis 4 Kohlenstoffatomen, wie Methoxy, Aethoxy, Propyloxy, Isopropyloxy, Butyloxy, Isobutyloxy, sek.-Butyloxy und tert.-Butyloxy, Phenoxy, Alkanoylaminogruppen mit 1 bis 6 Kohlenstoffatomen, wie Acetylamino oder Propionylamino, Benzoylamino, Aminogruppen, wie —$NH_2$, Methylamino, Aethylamino, Dimethylamino, Diäthylamino, Cyanäthylamino, Hydroxyäthylamino, Dihydroxyäthylamino, Cyclohexylamino, Benzylamino und Phenylamino, Carbonsäureestergruppen, wie Methoxycarbonyl und Aethoxycarbonyl, Trifluormethyl, Nitro, Cyan, Acetyl, Methylsulfonyl, Carbamoyl, Sulfamoyl, Ureido, Hydroxy, Carboxy, Sulfo, Sulfomethyl und Halogen, wie Fluor, Chlor und Brom, sowie faserreaktive Reste.

Vorzugsweise ist K der Rest eines Amino- oder Alkoxybenzols, Amino- oder Alkoxynaphthalins, Naphthols, Aminonaphthols, Pyrazolons, Aminopyrazols, Pyridons, Pyrimidins, Indols, Naphthylimidazols, Diphenylamins, Pyrazolo [2,3-a]pyrimidins, Tetrahydrochinolins oder Acetessigsäureamids, wobei die oben genannten Reste weitersubstituiert sein können.

Bedeuten $R_1$, X und Y in Formel (1) einen $C_{1-4}$-Alkylrest, so kommt unabhängig voneinander beispielsweise ein Methyl-, Aethyl-, Propyl-, Isopropyl-, Butyl-, sec.-Butyl-, Isobutyl- oder tert.-Butylrest in Betracht.

Falls $R_2$ ein Monosulfo-$C_{1-12}$-alkylrest ist, handelt es sich um einen geradkettigen oder verzweigten Monosulfoalkylrest, der weitersubstituiert sein kann, z. B. durch Halogen, wie Fluor, Chlor oder Brom, Hydroxy, Cyano, $C_{1-4}$-Alkoxy, wie Methoxy, Aethoxy, Propoxy, Isopropoxy, Butoxy, sek.-Butoxy, tert.-Butoxy oder Isobutoxy, und Alkanoylgruppen mit 1 bis 6 Kohlenstoffatomen, wie die Acetyl- oder Propionylgruppe, und die Benzoylgruppe. Als Beispiele für $R_2$ als Monosulfo-$C_{1-12}$-alkylrest seien genannt : Sulfomethyl, β-Sulfoäthyl, γ-Sulfopropyl und o-Sulfobutyl. Vorzugsweise ist $R_2$ als Monosulfoalkylrest ein β-Sulfoäthylrest.

Bedeutet $R_2$ einen Rest der Formel

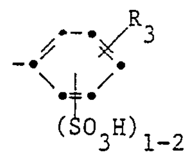

(2),

so ist $R_3$ Wasserstoff, $C_{1-4}$-Alkyl, wie z. B. Methyl, Aethyl, Propyl, Isopropyl, Butyl, Isobutyl, sec.-Butyl und tert.-Butyl, $C_{1-4}$-Alkoxy, wie z. B. Methoxy, Aethoxy, Propoxy, Isopropoxy, Butoxy, Isobutoxy, sec.-Butoxy und tert.-Butoxy, Halogen, wie z. B. Fluor, Chlor und Brom, $C_{2-6}$-Alkanoylamino, wie z. B. Acetylamino, Propionylamino und Butyrylamino oder Carboxy ist. Als Beispiele seien genannt : Der 2-, 3- oder 4-Sulfophenyl-, 2,5-Disulfophenyl-, 4-Acetylamino-3-sulfophenyl-, 2-Chlor-5-sulfophenyl-, 2-Carboxy-4-sulfophenyl-, 2-Methyl-4-sulfophenyl-, 2-Methoxy-5-sulfophenyl-, 4-Methyl-3-sulfophenyl- und 4-Chlor-3-sulfophenylrest.

Bedeutet $R_2$ einen Rest der Formel

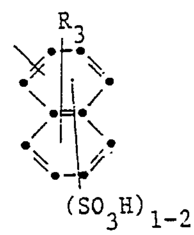

(3),

so hat $R_3$ die unter Formel (2) angegebene Bedeutung. Als Beispiele seien genannt : Der 1-Sulfonaphthyl-(2)-, 6-Sulfonaphthyl-(2) und 1,5-Disulfonaphthyl-(2)-Rest.

Bedeuten X und Y einen $C_{1-4}$-Alkoxyrest, so kommt unabhängig voneinander beispielsweise ein Methoxy-, Aethoxy-, Propoxy-, Isopropoxy-, Butoxy-, Isobutoxy-, sec.-Butoxy- oder tert.-Butoxyrest in Betracht.

Bedeuten X und Y Halogen, so kommt unabhängig voneinander beispielsweise ein Fluor-, Chlor- oder Bromatom in Betracht.

Bedeuten X und Y $C_{2-6}$-Alkanoylamino, so kommt unabhängig voneinander beispielsweise ein Acetylamino- oder Propionylaminorest in Betracht.

Bedeuten X und Y einen Phenylrest, so kann dieser weitersubstituiert sein, beispielsweise durch $C_{1-4}$-Alkylgruppen, wie Methyl, $C_{1-4}$-Alkoxygruppen wie Methoxy und Aethoxy, Halogen, wie Fluor, Chlor oder Brom, Alkanoylaminogruppen mit 1 bis 6 Kohlenstoffatomen, wie Acetylamino, Hydroxy und Carboxy.

Bedeuten X und Y einen Phenoxyrest, so kommt unabhängig voneinander vorzugsweise ein Phenoxyrest in Betracht, der im Phenylteil wie oben angegeben weitersubstituiert sein kann.

Bevorzugt sind Monoazofarbstoffe der Formel (1), worin Y Wasserstoff, Chlor oder Methoxy ist.

In den bevorzugten Monoazofarbstoffen der Formel (1) ist X in p-Stellung zur —$SO_2$-Gruppe an den Phenylring gebunden.

Die Monoazofarbstoffe der Formel (1) können in der Kupplungskomponente K druch faserreaktive Gruppen substituiert sein.

Geeignete Faserreaktivgruppen sind z. B. solche der aliphatischen Reihe, wie Acryloyl, Mono-, Di- oder Trichlor- bzw. Mono-, Di- oder Tribromacryloyl oder -metacryloyl, wie —CO—CH = CH—Cl, —CO—CCl=$CH_2$, —CO—CH=CHBr, —COCBr=$CH_2$, —CO—CBr=CHBr, —CO—CCl=CH—$CH_3$, ferner —CO—CCl=CH—COOH, —CO—CH=CCl—COOH, 3-Chlorpropionyl, 3-Phenylsulfonylpropionyl, 3-Methylsulfonylpropionyl, β-Sulfatoäthylaminosulfonyl, Vinylsulfonyl, β-Chloräthylsulfonyl, β-Sulfatoäthylsulfonyl, β-Methylsulfonyläthylsulfonyl, β-Phenylsulfonyläthylsulfonyl, 2-Fluor-2-chlor-3,3-difluorcyclobutan-1-carbonyl, 2,2,3,3-Tetrafluorcyclobutan-carbonyl-1 oder -sulfonyl-1, β-(2,2,3,3-Tetrafluorcyclobutyl-1)-acryloyl, α- oder β-Alkyl- oder -Arylsulfonyl-acryloyl, wie α- oder β-Methylsulfonylacryloyl.

Besonders für synthetisches Polyamid und für Wolle geeignete Reaktivreste sind : Chloracetyl, Bromacetyl, α,β-Dichlor- oder α,β-Dibrompropionyl, α-Chlor- oder α-Bromacryloyl, 2,4-Difluor-5-chlorpyrimidyl-6, 2,4,6-Trifluorpyrimidyl-5, 2,4-Dichlor-5-methylsulfonylpyrimidinyl-6, 2,4-Difluor-5-methyl-sulfonylpyrimidyl-6, 2,4-Difluortriazinyl-6, sowie Fluortriazinylreste der Formel

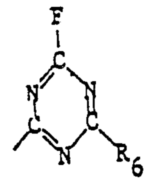

worin $R_6$ eine gegebenenfalls substituierte Aminogruppe oder eine gegebenenfalls verätherte Oxy- oder Thiogruppe bedeutet, wie z. B. die $NH_2$-Gruppe, eine mit $C_1$-$C_4$-Alkylresten mono- oder disubstituierte Aminogruppe, eine $C_1$-$C_4$-Alkoxygruppe, eine $C_1$-$C_4$-Alkylmercaptogruppe, Arylamino, insbesondere Phenylamino, oder mit Methyl, Methoxy, Chlor und vor allem Sulfo substituiertes Phenylamino, Phenoxy, Mono- oder Disulfophenoxy etc., sowie die entsprechenden Chlortriazinylreste.

Ausgangsprodukte zur Einführung solcher Triazinreste sind z. B. 2,4-Difluor- oder 2,4-Dichlor-6-aminotriazin, 2,4-Difluor- oder 2,4-Dichlor-6-methylaminotriazin, 2,4-Difluor- oder 2,4-Dichlor-6-äthylaminotriazin, 2,4-Difluor- oder 2,4-Dichlor-6-phenylaminotriazin, 2,4-Difluor- oder 2,4-Dichlor-6-(2′-3′- oder 4′-sulfophenyl)-aminotriazin, 2,4-Difluor- oder 2,4-Dichlor-6-(2′-4′ oder 3′,4′- oder 2′,5′- oder 4′,5′-disulfophenyl)-aminotriazin, 2,4-Difluor- oder 2,4-Dichlor-6-dimethylaminotriazin, 2,4-Difluor- oder 2,4-Dichlor-6-methoxytriazin, 2,4-Difluor- oder 2,4-Dichlor-6-(β-methoxyäthoxy)-triazin, 2,4-Difluor- oder 2,4-Dichlor-6-methylmercaptotriazin und 2,4-Difluor- oder 2,4-Dichlor-6-phenylmercaptotriazin sowie 2,4,6-Trifluor- oder 2,4,6-Trichlortriazin.

Bevorzugt sind die Monoazofarbstoffe der Formel (1), worin K ein N-$C_{1-4}$ Alkylamino- oder N,N-Di-$C_{1-4}$-alkylaminobenzolrest, der im Benzolring durch Methyl, Chlor, Acetylamino und Benzoylamino substituiert sein kann, und worin die N-Alkylreste unabhängig voneinander durch Hydroxy, Chlor, Cyano, Phenyl oder Sulfophenyl substituiert sein können, ein Methoxybenzolrest, ein Methoxynaphthalinrest, ein Hydroxynaphthalinrest, der durch Sulfo substituiert sein kann, ein Hydroxynaphthalinrest, der durch Sulfo substituiert sein kann, ein Aminonaphtholrest, der im Naphthalinkern durch Sulfo substituiert sein kann, und worin die Aminogruppe durch Methyl, α,β-Dibrompropionyl, α,β-Dibrompropionylaminobenzoyl und 2,6-Difluor-5-chlorpyrimidylaminobenzoyl substituiert sein kann, ein 1-Phenyl-3-methylpyrazolonrest, der im Phenylrest durch Chlor und Methyl substituiert sein kann, ein 1-Phenyl-3-methylaminopyrazolrest, der im Phenylrest durch Chlor und Sulfo substituiert sein kann, ein 1-Aethyl-4-methyl-6-hydroxy-pyridon-(2)-rest, der in 3-Stellung durch Carbamoyl substituiert sein kann, ein 2,4,6-Triaminopyrimidinrest, ein 2-Methylindol- oder 1-Aethyl-2-methylindolrest, ein 1-Chlorphenylamino-2-methyl- oder 2-pentyl-naphthimidazolrest, der im Naphthalinkern durch Hydroxy und Sulfo substituiert ist, ein N-Methyl- oder N-Aethyl-1,2,3,4-tetrahydrochinolinrest, oder ein 2,5-Dimethyl-7-amino-pyrazolo[2,3-a]pyrimidinrest und R eine

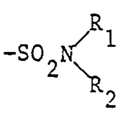 , 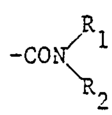 ,

—CO—$R_2$ oder —$SO_2$—O—$R_2$-Gruppe ist, wobei $R_1$ Wasserstoff oder Methyl und $R_2$ β-Sulfoäthyl, 2-, 3- oder 4-Sulfophenyl, wobei der Phenylring durch Methyl, Methoxy, Chlor, Acetyl-amino und Carboxy substituiert sein kann, 2,5-Disulfophenyl oder Monosulfo- oder Disulfonaphthyl ist, und worin X Wasserstoff, Methyl, Chlor, Aethoxy, Acetylamino oder Phenoxy und Y Wasserstoff, Chlor oder Methoxy ist.

Besonders bevorzugt sind die Monoazofarbstoffe der Formel (1), worin K ein N,N-Di-$C_{1-4}$-alkylaminobenzolrest, der im Benzolkern durch Methyl, Chlor, Acetylamino und Benzoylamino substituiert sein kann, und worin die N-Alkylreste unabhängig voneinander durch Hydroxy, Chlor, Cyano, Phenyl oder Sulfophenyl substituiert sein können, ein 1-Phenyl-3-methyl-aminopyrazolrest, der im Phenylrest durch Chlor und Sulfo substituiert sein kann, oder ein 2-Methylindol- oder 1-Aethyl-2-methyl-indolrest ist und R, X und Y die oben angegebenen Bedeutungen haben.

Insbesondere bevorzugt sind die Monoazofarbstoffe der Formel (1), worin K ein N,N-Diäthyl-3-methylanilinrest, ein N-Aethyl-N-β-hydroxyäthyl-3-methylanilinrest, ein 1-Phenyl-3-methyl-5-aminopyrazolrest oder ein 2-Methylindolrest, R ein β-Sulfoäthylaminosulfonyl-, o-Sulfophenylaminosulfonyl-, p-Sulfophenylaminosulfonyl-, p-Sulfophenylaminocarbonyl- oder p-Methyl-m-sulfobenzoylrest, X Methyl oder Chlor und Y Wasserstoff ist.

Gegenstand der Erfindung ist ferner ein Verfahren zur Herstellung der Monoazofarbstoffe der Formel (1). Das Verfahren ist dadurch gekennzeichnet, dass man eine Diazokomponente der Formel

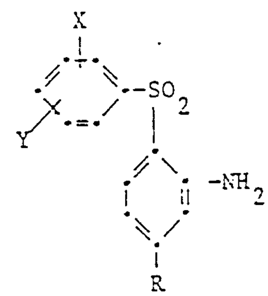

(4)

diazotiert und auf eine Kupplungskomponente der Formel

H—K (5)

kuppelt, wobei K, R, X und Y in den Formeln (4) und (5) die unter Formel (1) angegebenen Bedeutungen haben.

Die Diazotierung der Diazokomponente der Formel (4) erfolgt in der Regel durch Einwirkung salpetriger Säure in wässrig-mineralsaurer Lösung bei tiefer Temperatur, die Kupplung auf die Kupplungskomponente der Formel (5) bei sauren, neutralen bis alkalischen pH-Werten.

Gegebenenfalls kann eine freie Aminogruppe im Rest K nach der Kupplung mit einem Acylierungs- oder Alkylierungsmittel in eine Acylamino- oder Alkylaminogruppe umgewandelt werden, und ebenso kann eine Hydroxygruppe im Rest K durch Alkylierung in eine Alkoxygruppe übergeführt werden.

Die Azofarbstoffe der Formel (1), die einen faserreaktiven Rest enthalten, werden hergestellt, indem man eine Diazokomponente der Formel (4), eine Kupplungskomponente der Formel (5), die eine acylierbare Amino- oder Hydroxygruppe enthält, und ein Acylierungsmittel, das einen faserreaktiven Rest enthält, in beliebiger Reihenfolge miteinander umsetzt.

Vorzugsweise werden als Kupplungskomponenten, die eine acylierbare Gruppe enthalten, solche der Benzol- oder Naphthalinreihe verwendet.

Bevorzugte Verfahrensvarianten sind dadurch gekennzeichnet, dass man

a) eine Kupplungskomponente der Formel (5) verwendet, worin K der Rest eines Amino- oder Alkoxybenzols, Amino- oder Alkoxynaphthalins, Naphthols, Aminonaphthols, Pyrazolons, Aminopyrazols, Pyridons, Pyrimidins, Indols, Naphthimidazols, Diphenylamins, Pyrazolo[2,3-a]pyrimidins, Tetrahydrochinolins oder Acetessigsäureamids ist, wobei die oben genannten Reste weitersubstituiert sein können,

b) eine Diazokomponente der Formel (4) verwendet, worin $R_2$ einen unsubstituierten Monosulfo-$C_{1-12}$-alkylrest, einen Rest der Formel

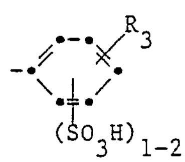

(2)

oder einen Rest der Formel

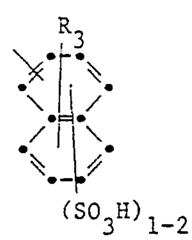

(3)

bedeutet, worin $R_3$ in den Formeln (2) und (3) Wasserstoff, $C_{1-4}$-Alkyl, $C_{1-4}$-Alkoxy, Halogen, $C_{2-6}$-Alkanoylamino oder Carboxy ist,

c) eine Diazokomponente der Formel (4) verwendet, worin Y Wasserstoff, Chlor oder Methoxy ist,

d) eine Diazokomponente der Formel (4) verwendet, worin X in p-Stellung zur —$SO_2$-Gruppe an den Phenylring gebunden ist.

Die besonders bevorzugten Monoazofarbstoffe der Formel (1) werden hergestellt, indem man eine Diazokomponente der Formel (4), worin R eine

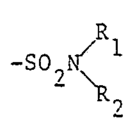 , 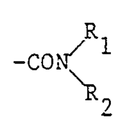 ,

—CO—$R_2$ oder —$SO_2$—O—$R_2$-Gruppe ist, wobei $R_1$ Wasserstoff oder Methyl und $R_2$ β-Sulfoäthyl, 2-, 3- oder 4-Sulfophenyl, wobei der Phenylring durch Methyl, Methoxy, Chlor, Acetylamino und Carboxy substituiert sein kann, 2,5-Disulfophenyl oder Monosulfo- oder Disulfonaphthyl ist, und worin X Wasserstoff, Methyl, Chlor, Aethoxy, Acetylamino oder Phenoxy, und Y Wasserstoff, Chlor oder Methoxy ist, diazotiert und auf eine Kupplungskomponente der Formel (5) kuppelt, worin K ein N-$C_{1-4}$-Alkylamino- oder N,N-Di-$C_{1-4}$-alkylamino-benzolrest, der im Benzolring durch Methyl, Chlor, Acetylamino und Benzoylamino substituiert sein kann, und worin die N-Alkylreste unabhängig voneinander durch Hydroxy, Chlor, Cyano, Phenyl oder Sulfophenyl substituiert sein können, ein Methoxybenzolrest, ein Methoxynaphthalinrest, ein Aminonaphthalinrest, der durch Sulfo substituiert sein kann, ein Hydroxynaphthalinrest, der durch Sulfo substituiert sein kann, ein Aminonaphtholrest, der im Naphthalinkern durch Sulfo substituiert sein kann, und worin die Aminogruppe durch Methyl, α,β-Dibrompropionyl, α,β-Dibrompropionylaminobenzoyl und 2,6-Difluor-5-chlorpyrimidylaminobenzoyl substituiert sein kann, ein 1-Phenyl-3-methyl-pyrazolonrest, der im Phenylrest durch Chlor und Methyl substituiert sein kann, ein 1-Phenyl-3-methyl-aminopyrazolrest, der im Phenylrest durch Chlor und Sulfo substituiert sein kann, ein 1-Aethyl-4-methyl-6-hydroxy-pyridon-(2)-rest, der in 3-Stellung durch Carbamoyl substituiert sein kann, ein 2,4,6-Triaminopyrimidinrest, ein 2-Methylindol- oder 1-Aethyl-2-methylindolrest, ein 1-Chlorphenylamino-2-methyl- oder 2-pentyl-naphthimidazolrest, der im Naphthalinkern durch Hydroxy und Sulfo substituiert ist, ein N-Methyl- oder N-Aethyl-1,2,3,4-tetrahydrochinolinrest, oder ein 2,5-Dimethyl-7-amino-pyrazolo[2,3-a]pyrimidinrest ist.

Insbesondere verwendet man eine Kupplungskomponente der Formel (5), worin K ein N,N-Di-$C_{1-4}$-alkylaminobenzolrest, der im Benzolkern durch Methyl, Chlor, Acetylamino und Benzoylamino substituiert sein kann und worin die N-Alkylreste unabhängig voneinander durch Hydroxy, Chlor, Cyano, Phenyl oder Sulfophenyl substituiert sein können, ein 1-Phenyl-3-methyl-aminopyrazolrest, der im Phenylrest durch Chlor und Sulfo substituiert sein kann, oder ein 2-Methylindol- oder 1-Aethyl-2-methyl-indolrest ist.

Die ganz besonders bevorzugten Monoazofarbstoffe der Formel (1) werden hergestellt, indem man eine Diazokomponente der Formel (4), worin R ein β-Sulfoäthylaminosulfonyl-, o-Sulfophenylaminosulfonyl-, p-Sulfophenylaminosulfonyl-, p-Sulfophenylaminocarbonyl- oder p-Methyl-m-sulfobenzoylrest, X Methyl oder Chlor und Y Wasserstoff ist, diazotiert und auf eine Kupplungskomponente der Formel (5), worin K ein N,N-Diäthyl-3-methylanilinrest, ein N-Methyl-N-β-hydroxyäthyl-3-methyl-anilinrest, ein 1-Phenyl-3-methyl-5-aminopyrazolrest oder ein 2-Methyl-indolrest ist, kuppelt.

Aus der grossen Zahl möglicher Kupplungskomponenten seien folgende als Beispiele genannt :

Anilin, Dimethylanilin, Diäthylanilin, 3-Methyl-dimethylanilin, 3-Methyl-dibutylanilin, 3-Methyl-diäthylanilin, 3-Acetylamino- oder 3-Methoxycarbonylamino- oder 3-Ureido-dimethylanilin, 3-Methyl-6-methoxy-diäthylanilin, 3-Methyl-N-äthyl-N-butylanilin, 2,5-Dimethoxy-diäthylanilin, N-Aethyl-N-benzylanilin, N-Aethyl-N-(β-cyanäthyl)-anilin, N-Aethyl-N-(β-hydroxyäthyl)-anilin, N-Aethyl-N-(β-acetoxy-äthyl)-anilin, N,N-Dibutylanilin, 3-Acetylamino-N,N-diäthylanilin, N-Methyl-N-(β-cyanäthyl)-anilin, 3-Methyl-N,N-di-(β-cyanäthyl)-anilin, 3-Chlor-N,N-dimethylanilin, 3-Methyl-N-äthyl-N-benzylanilin, N,N-Di-n-propylanilin, 3-Acetylamino-N,N-di-(β-hydroxyäthyl)-anilin, 3-Methyl-N,N-di-(β-acetoxyäthyl)-lin, 3-Methyl-N-äthyl-N-(3'-sulfobenzyl)-anilin, N-Aethyl-N-(β-chloräthyl)-anilin, 2-Methoxy-5-acetylamino-N-benzylanilin, 2-Methoxy-5-acetylamino-N-(β-acetoxyäthyl)-N-benzylanilin, 3-Methyl-N,N-dibutylanilin, 3-Methyl-N-äthyl-N-(β-cyanäthyl)-anilin, 2-Methyl-5-acetylamino-N,N-dimethylanilin, 2-Chlor-5-acetylamino-N,N-dimethylanilin, 2-Chlor-5-acetylamino-N-(γ-phenoxy-β-hydroxy-n-propyl)-anilin, 3-Ureidoanilin, N-Aethyl-N-(β-hydroxyäthyl)-anilin, N-Aethyl-N-(3'-sulfobenzyl)-anilin, 3-Methyl-N-äthyl-(β-sulfoäthyl)-anilin, 3-Benzoylamino-N,N-diäthylanilin, 3-(p-Tolylsulfamoyl)-N,N-diäthylanilin, 3-(p-Chlorbenzoylamino)-N,N-diäthylanilin, 3-Methoxy-N,N-diäthylanilin, 3-Methyl-N,N-di-(β-hydroxyäthyl)-anilin, 3-Methyl-6-methoxy-N,N-di-(β-hydroxyäthyl)-anilin, 3-Acetylaminoanilin, 3-Methyl-N-äthyl-N-phenäthylanilin, N,N-Di-(β-cyan- oder-hydroxyäthyl)-anilin, 3-Acetylamino-N,N-di-(β-cyanäthyl)-anilin, 3-Methyl-N-äthyl-N-(β-hydroxyäthyl)-anilin, 3-Methyl-N-äthyl-N(β-hydroxyäthyl)-anilin, N-(β-cyanäthyl)-anilin, N-Methyl-N-benzylanilin, Phenol, 3-Methylphenol, Methoxybenzol, 3-Aethoxytoluol, 1-Hydroxy-4-methoxybenzol, 1-Hydroxy-4-tert.-butylbenzol, 1-Hydroxy-7-amino-3-sulfonaphthalin, 1-Hydroxy-7-methylamino- oder 7-phenylamino-3-sulfonaphthalin, 2-Aminonaphthalin, 2-Amino-6-sulfonaphthalin, 2-Amino-5-acetylaminomethylnaphthalin, β-Naphthol, 2-Amino-5-methylaminosulfonylnaphthalin, 1-Hydroxy-8-amino-3,6- oder -3,5-disulfonaphthalin, 1-Hydroxy-8-benzoylamino-3,6- oder -3,5-disulfonaphthalin, 1-Hydroxy-8-ureido-3,6- oder -3,5-disulfonaphthalin, 1-Hydroxy-8-acetylamino-3,6- oder -3,5-disulfonaphthalin, 1-[3'-Chlorphenyl)-3-methylpyrazolon-5, 1-(2'-Chlor-6'-methylphenyl)-3-methylpyrazolon-5, 1-Phenyl-3-methylpyrazolon-5, 1-(2'-3'- oder 4'-sulfophenyl)-3-methylpyrazolon-5, 1-(2'-Chlor-4'- oder 5'-Sulfophenyl)-3-methylpyrazolon-5, 1-(3'- oder 4'-Dibrompropionylamino)-benzoylamino-8-hydroxy-3,6- oder -4,6-disulfonaphthalin, 2-α,β-Dibrompropionyl-amino-1-hydroxy-6-sulfonaphthalin, 3-α,β-Dibrompropionyl-N-methyl-amino-8-hydroxy-6-sulfonaphthalin, 1-[3'-(2",4"-Difluor-5'-chlorpyrimidyl-amino)-benzoylamino]-8-hydroxy-4,6-disulfonaphthalin, 1-(2'-Methyl-4'-sulfophenyl)-3-methylpyrazolon-5, 1-[4',8'-Disulfonaphthyl-(2)]-3-methylpyrazolon-5, 1-[5'-7'-Disulfonaphthalin-(2)]-3-methylpyrazolon-5, 1-(2'-Chlor-5'-sulfophenyl)-3-methyl-5-aminopyrazol, 1-(2'-Chlor-4'-sulfophenyl)-3-methyl-5-aminopyrazol, 1-(3'- oder 4'-Sulfophenyl)-3-methyl-5-aminopyrazol, 1-Aethyl-3-cyano-4-methyl-6-hydroxy-pyridon-2, 1-Aethyl-3-carbamoyl-4-methyl-6-hydroxy-pyridon-2, 1-Aethyl-4-methyl-6-hydroxy-pyridon-2, 1-Aethyl-4-methyl-3-methylsulfonyl-6-hydroxypyridon-2, 2-Methylindol, 2-Phenylindol, 1-Methyl-2-phenylindol, 1-Octyl-2-methylindol, 2,4,6-Triaminopyrimidin, N-Methyl-N,N-diphenylamin, Acetessigsäureanilid, 1-(4'-Methylphenyl)-3-methylpyrazolon-5, 1-Aethyl-2-methylindol, 1-Phenyl-3-methyl-5-aminopyrazol. 7-Amino-2,5-dimethyl-pyrazol[2,3-a]pyrimidin, 1-Methoxynaphthalin, 1-Hydroxy-4-sulfonaphthalin, 1-(2'-Chlorphenylamino)-9-hydroxy-2-methyl-1H-naphtho(1.2-d)imidazol-7-sulfonsäure, 1-(2'-Chlorphenylamino)-9-hydroxy-2-pentyl-1H-naphtho(1.2-d)imidazol-7-sulfonsäure, N-Methyl- oder N-Aethyl-1,2,3,4-tetrahydrochinolin, 3-Methyl-N-äthyl-N-propylanilin.

Die vorliegende Erfindung betrifft ebenfalls die als Diazokomponenten verwendeten Verbindungen der Formel

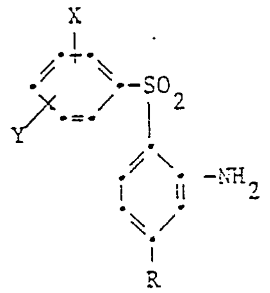

(4),

worin R, X und Y die unter Formel (1) angegebenen Bedeutungen haben.

Bevorzugt sind die Verbindungen der Formel (4), worin R eine

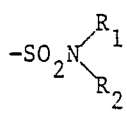 , 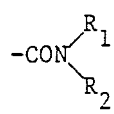 ,

$—CO—R_2$ oder $—SO_2—O—R_2$-Gruppe ist, wobei $R_1$ Wasserstoff oder $C_{1-4}$-Alkyl und $R_2$ einen unsubstituierten Monosulfo-$C_{1-12}$-alkylrest, einen Rest der Formel

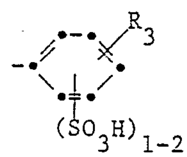

(2)

oder einen Rest der Formel

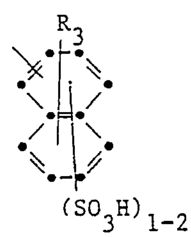

(3)

bedeutet, worin $R_3$ in den Formeln (2) und (3) Wasserstoff, $C_{1-4}$-Alkyl, $C_{1-4}$-Alkoxy, Halogen, $C_{2-6}$-Alkanoylamino oder Carboxy ist und X und Y die unter Formel (1) angegebenen Bedeutungen haben.

Ebenfalls bevorzugt sind Verbindungen der Formel (4), worin

a) Y Wasserstoff, Chlor oder Methoxy ist, und

b) X in p-Stellung zur $—SO_2$-Gruppe an den Phenylring gebunden ist.

Besonders bevorzugt sind die Verbindungen der Formel (4), worin R eine

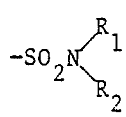 , 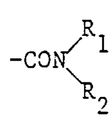 ,

$—CO—R_2$ oder $—SO_2—O—R_2$-Gruppe ist, wobei $R_1$ Wasserstoff oder Methyl und $R_2$ β-Sulfoäthyl, 2-, 3- oder 4-Sulfophenyl, wobei der Phenylring durch Methyl, Methoxy, Chlor, Acetylamino und Carboxy substituiert sein kann, 2,5-Disulfophenyl oder Monosulfo- oder Disulfonaphthyl ist, und worin X Wasserstoff, Methyl, Chlor, Aethoxy, Acetylamino oder Phenoxy und Y Wasserstoff, Chlor oder Methoxy ist.

Insbesondere bevorzugt sind die Verbindungen der Formel (4), worin R ein β-Sulfoäthylaminosulfo-

nyl-, o-Sulfophenylaminosulfonyl-, p-Sulfophenylaminosulfonyl-, p-Sulfophenylaminocarbonyl- oder p-Methyl-m-sulfobenzoylrest, X Methyl oder Chlor und Y Wasserstoff ist.

Die Verbindungen der Formel (4) werden hergestellt, indem man eine Verbindung der Formel

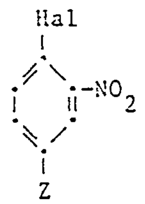

(6),

worin Z eine $—SO_2—$Hal oder —CO—Hal Gruppe ist und Hal Halogen bedeutet, mit einer Verbindung der Formel

$$[H(R_1)N, HO, H]—R_2 \qquad (7),$$

worin $R_1$ und $R_2$ die unter Formel (4) angegebenen Bedeutungen haben, zu einer Verbindung der Formel

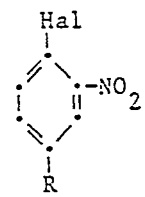

(8),

worin R die unter Formel (4) angegebene Bedeutung hat und Hal Halogen ist, umsetzt, und die Verbindung der Formel (8) mit einer Verbindung der Formel

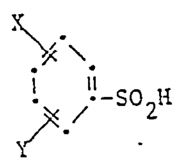

(9),

worin X und Y die unter Formel (4) angegebenen Bedeutungen haben, kondensiert und anschliessend die $NO_2$-Gruppe reduziert.

Eine Verfahrensvariante zur Herstellung der Verbindungen der Formel

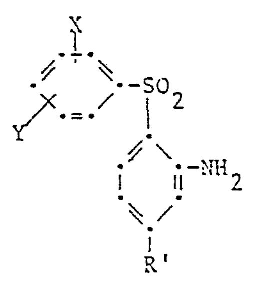

(10),

worin R' eine

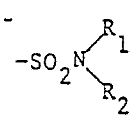

oder

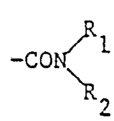

Gruppe ist, und $R_1$, $R_2$, X und Y die unter Formel (4) angegebenen Bedeutungen haben, ist dadurch gekennzeichnet, dass man eine Verbindung der Formel

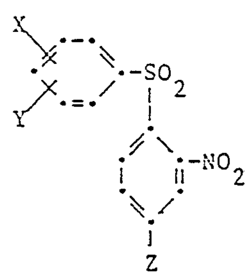

(11),

worin Z eine $-SO_2-Hal$ oder $-CO-Hal$-Gruppe ist und Hal Halogen bedeutet, mit einem Amin der Formel

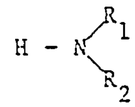

(12)

umsetzt und anschliessend die Nitrogruppe reduziert, wobei $R_1$, $R_2$, X und Y in den Formeln (11) und (12) die unter Formel (10) angegebenen Bedeutungen haben.

Die Verfahrensweisen zur Herstellung der Verbindungen der Formel (4) sind an sich bekannt. Die Umsetzungen erfolgen bei Temperaturen zwischen 0 und 150 °C, gegebenenfalls unter Druck in wässriger Lösung oder Gemischen aus organischen Lösungsmitteln, wie z. B. Aceton und Wasser und bei pH-Werten zwischen 7 und 10. Die Reduktion der Nitrogruppe erfolgt ebenfalls nach an sich bekannten Methoden, wie z. B. in Eisessig in Gegenwart von Eisenpulver.

Als Ausgangsverbindungen der Formel (6) seien beispielsweise genannt : 4-Chlor-3-nitrobenzoylchlorid, 4-Chlor-3-nitrobenzol-1-sulfonsäurechlorid.

Als Ausgangsverbindungen der Formel (7) seien beispielsweise genannt : 2-, 3- oder 4-Sulfophenylanilin, Taurin, N-Methyltaurin, 2-Aminonaphthalin-6-sulfonsäure, 2-Aminonaphthalin-1-sulfonsäure, 2-Aminonaphthalin-1,5-disulfonsäure, 2,5-Disulfoanilin, 4-Acetylamino-3-sulfoanilin, 2-Chlor-5-sulfoanilin, 2-Carboxy-4-sulfoanilin, 2-Methyl-4-sulfoanilin, 2-Methoxy-5-sulfoanilin, 4-Methyl-3-sulfoanilin, 4-Sulfophenol, 2-Sulfotoluol, 2-Chlorbenzolsulfonsäure.

Als Ausgangsverbindungen der Formel (9) seien beispielsweise genannt : 4-Chlorbenzolsulfinsäure, Benzolsulfinsäure, 4-Aethoxybenzolsulfinsäure, 4-Acetylaminobenzolsulfinsäure, 4-Phenoxybenzolsulfinsäure, 4-Brombenzolsulfinsäure, 4-Jodbenzolsulfinsäure, 2-Methyl-, 3-Methyl- oder 4-Methylbenzolsulfinsäure, 2,3-Dimethylbenzolsulfinsäure, 3,4-Dimethylbenzolsulfinsäure, 2,4-, 2,5- oder 3,5-Dimethylbenzolsulfinsäure, 2-Methyl-5-isopropylbenzolsulfinsäure, 2-Methoxy- oder 2-Aethoxybenzolsulfinsäure, 4-Methoxybenzolsulfinsäure, 2-Methoxy-5-methylbenzolsulfinsäure, 2-Brom-5-methoxybenzolsulfinsäure, 2-Methyl-4-chlorbenzolsulfinsäure.

Als Ausgangsverbindungen der Formel (11) seien beispielsweise genannt : 4'-Chlor-, 4'-Methyl-, 4'-Aethoxy-, 4'-Acetylamino-, 4'-H- oder 4'-Phenoxy-2-nitro-1,1'-diphenylsulfon-4-sulfonsäure- oder 4-carbonsäurechlorid.

Als Ausgangsverbindungen der Formel (12) kommen beispielsweise die für Formel (7) genannten Amine in Betracht.

Als Beispiele für die Verbindungen der Formel (4) seien genannt :

4'-Chlor-, 4'-Methyl-, 4'-Aethoxy-, 4'-Acetylamino- oder 4'-Phenoxy-2-amino-1,1'-diphenylsulfon-4-sulfonsäure- oder -carbonsäure-N-β-sulfoäthylamid,

4'-Chlor-, 4'-Methyl-, 4'-Aethoxy-, 4'-Acetylamino- oder 4'-Phenoxy-2-amino-1,1'-diphenylsulfon-4-sulfonsäure- oder -carbonsäure-N-2"-, 3"- oder 4"-sulfoanilid,

4'-Chlor-, 4'-Methyl-, 4'-Aethoxy-, 4'-Acetylamino- oder 4'-Phenoxy-2-amino-1,1'-diphenylsulfon-4-sulfonsäure- oder -carbonsäure-N-1"-sulfo-, 6"-sulfo- oder 1",5"-disulfonaphthyl-(2)-amid,

2-Amino-1,1'-diphenylsulfon-4-sulfonsäure- oder -carbonsäure-N-methyl-N-β-sulfoäthylamid,

2-Amino-1,1'-diphenylsulfon-4-sulfonsäure- oder -carbonsäure-2"-, 3"- oder 4"-sulfoanilid,

2-Amino-1,1'-diphenylsulfon-4-sulfonsäure- oder -carbonsäure-N-β-sulfoäthylamid,

2-Amino-1,1'-diphenylsulfon-4-sulfonsäure- oder -carbonsäure-N-1"-sulfo-, 6"-sulfo- oder 1",5"-disulfonaphthyl-(2)-amid,

2-Amino-1,1'-diphenylsulfon-4-sulfonsäure-4"-acetylamino-3"-sulfoanilid,

4'-Methyl-2-amino-1,1'-diphenylsulfon-4-sulfonsäure-2"-chlor-5"-sulfo-anilid,

2-Amino-1,1'-diphenylsulfon-4-sulfonsäure-2"-chlor-5"-sulfo-anilid,

2-Amino-1,1'-diphenylsulfon-4-sulfonsäure-2"-carboxy-4"-sulfo-anilid,

4'-Chlor-2-amino-1,1'-diphenylsulfon-4-sulfonsäure-2",5"-disulfo-anilid,

4'-Methyl-2-amino-1,1'-diphenylsulfon-4-sulfonsäure-2"-carboxy-4"-sulfo-anilid,

4'-Methyl-2-amino-1,1'-diphenylsulfon-4-sulfonsäure-2"-methyl-4"-sulfo-anilid,

2-Amino-1,1'-diphenylsulfon-4-sulfonsäure-2"-methyl-4"-sulfo-anilid,

4'-Methyl-2-amino-1,1'-diphenylsulfon-4-sulfonsäure-2"-methoxy-5"-sulfo-anilid,

2-Amino-1,1'-diphenylsulfon-4-sulfonsäure-2"-methoxy-5"-sulfo-anilid,

4'-Methyl-2'-methoxy-2-amino-1,1'-diphenylsulfon-4-sulfonsäure-3"-sulfo-anilid,

2'-Methoxy-2-amino-1,1'-diphenylsulfon-4-sulfonsäure-3"-sulfo-anilid,

4'-Chlor- oder 4'-Methyl-2-amino-1,1'-diphenylsulfon-4-carbonsäure-(2"-Chlor-5"-sulfo-, 2"-methyl-4"-sulfo-, 2"-methoxy-5"-sulfo- oder 4"-acetylamino-3"-sulfo)-anilid,

2-Amino-1,1'-diphenylsulfon-4-carbonsäure-(2"-chlor-5"-sulfo-, 2"-methyl-4"-sulfo-, 2"-methoxy-5"-sulfo- oder 4"-acetylamino-3"-sulfo)-anilid,

4'-Methyl-2-amino-1,1'-diphenylsulfon-4-sulfonsäure-4"-sulfophenylester,

4'-Acetylamino-2-amino-1,1'-diphenylsulfon-4-sulfonsäure-4"-sulfophenylester,

4'-Chlor- oder 4'-Methyl-2-aminc-1,1'-diphenylsulfon-4-(4"-methyl-3"-sulfo- oder 4"-chlor-3"-sulfo)-phenylcarbonyl,

2-Amino-1,1'-diphenylsulfon-4-(4"-methyl-3"-sulfo- oder 4"-chlor-3"-sulfo)-phenylcarbonyl,

4'-Brom-, 4'-Jod-, 2'-Methyl-, 3'-Methyl-, 2',3'-Dimethyl-, 3',4-Dimethyl-, 2',4'-Dimethyl-, 2',5'-Dimethyl- oder 3',5'-Dimethyl-2-amino-1,1'-diphenylsulfon-4-sulfonsäure- oder -carbonsäure-(N-β-sulfoäthylamid oder N-2"-, 3"- oder 4"-sulfoanilid).

2'-Methoxy-, 2'-Aethoxy-, 4'-Methoxy-, 2'-Methoxy-5'-methyl-, 2'-Brom-5'-methoxy- oder 2'-Methyl-4'-chlor-2-amino-1,1'-diphenylsulfon-4-sulfonsäure- oder -carbonsäure-(2"-chlor-5"-sulfo-, 2"-methyl-4"-sulfo-, 2"-methoxy-5"-sulfo- oder 4"-acetylamino-3"-sulfo)-anilid.

Die erfindungsgemäss verwendeten Farbstoffe liegen entweder in der Form ihrer freien Sulfonsäure oder vorzugsweise als deren Salze vor.

Als Salze kommen beispielsweise die Alkali-, Erdalkali- oder Ammoniumsalze oder die Salze eines organischen Amins in Betracht. Als Beispiele seien die Natrium-, Lithium-, Kalium- oder Ammoniumsalze oder das Salz des Triäthanolamins genannt.

Die Farbstoffe der Formel (1) eignen sich zum Färben und Bedrucken von Amidgruppen enthaltenden Materialien, wie Textilfasern, Textilfäden und -geweben aus Wolle, Seide und Polyurethanfasern, insbesondere aber zum Färben und Bedrucken von synthetischem Polyamid, wobei die üblichen Färbeverfahren angewendet werden.

Sie zeichnen sich aus durch Brillanz und Farbstärke, gutes Auszieh- und Aufbauvermögen und sie ergeben Färbungen von allgemein guten Echtheiten, wie Reibechtheit, Säure- und Alkaliechtheit, Nassechtheiten, insbesondere Wasch-, Wasser-, Heisswasser- und Schweissechtheit und sie geben egale Färbungen. Insbesondere hervorzuheben ist die sehr gute Lichtechtheit und die sehr gute Beständigkeit gegen Formaldehyd.

Aus der US-A-2 551 887 ist ein Nachchromierungsfarbstoff bekannt, der jedoch nicht alle guten Eigenschaften der erfindungsgemässen Farbstoffe aufweist.

In den folgenden Beispielen stehen Teile für Gewichtsteile. Die Temperaturen sind Celsiusgrade. Die Beziehung zwischen Gewichtsteilen und Volumenteilen ist dieselbe wie diejenige zwischen Gramm und Kubikzentimeter.

## Beispiel 1

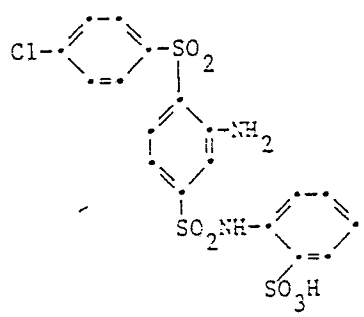

81,6 g Anilin-2-sulfonsäure (84,9 %ig) werden in 400 ml Wasser bei Raumtemperatur unter Zusatz von 10n Natronlauge bei pH 9 gelöst. Nach der Zugabe von 123,4 g 4-Chlor-3-nitrobenzol-1-sulfochlorid (82,9 %ig) lässt man die Reaktionsmischung unter Einhaltung von pH 9 mit 10n Natronlauge 28 Stunden bei Raumtemperatur nachrühren. Nach zusätzlichen 2 Stunden bei 45-50° wird die trübe Lösung bei pH 9 mit Aktivkohle klärfiltriert. Mit Salzsäure konz. wird auf pH 7 gestellt, das ausgefällte Produkt bei 30° filtriert und nach dem Waschen mit 250 ml Wasser bei 60-70° im Vakuumschrank getrocknet. Die Ausbeute beträgt 134,9 g.

131,5 g dieser Verbindung werden zusammen mit 96,0 g 4-Chlorbenzolsulfinsäure Na-Salz (68,8 %ig) in 1 000 ml Wasser verrührt und mit verdünnter Natronlauge auf pH 7,5 gestellt. Nach einer Verweilzeit von 15 Stunden bei 112-115° in einem Tantalautoklaven wird der vorliegende Brei bei 80° mit 300 ml Wasser verdünnt und filtriert. Der Trockengehalt des gut abgepressten Nutschkuchens beträgt 188 g.

187 g des vorliegenden Nitrodiphenylsulfons werden innerhalb von 25 Minuten bei 94-97° in 600 ml Wasser, enthaltend 64 g Eisenpulver und 4,5 ml Eisessig, eingetragen. Man lässt 20 Minuten bei Rückflusstemperatur nachrühren, fügt bei 90-80° vorsichtig 5,6 g Soda hinzu und filtriert den Eisenrückstand ab. Das Filtrat wird mit 10n Natronlauge auf pH 11,8 gestellt und mit Aktivkohle klärfiltriert. Nach Zugabe von Kochsalz wird 1 Stunde nachgerührt, die Ausfällung filtriert, mit Kochsalzlösung gewaschen und im Vakuum bei 60-70° getrocknet.

Man erhält in praktisch reiner Form 178 g der oben formulierten Verbindung.

(Siehe Figur Seite 11 f.)

Beispiel 2

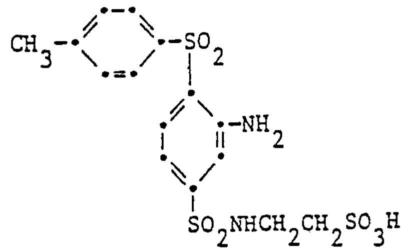

75 g Taurin werden in 600 ml Wasser verrührt und mit Natronlauge konz. auf pH 9,5 gestellt. Zu der auf 0° abgekühlten Lösung werden 203,5 g 4-Chlor-3-nitrobenzol-1-sulfochlorid (75,6 %ig) in 200 ml Aceton gelöst zugetropft. Man hält den oben eingestellten pH-Wert mit Natronlauge konstant und scheidet das ausgefallene Produkt nach Salzzugabe ab. Das gewaschene und getrocknete Produkt ergibt eine Ausbeute von 131,7 g.

131 g dieser Verbindung werden zusammen mit 57,6 g Toluol-4-sulfinsäure-Na-Salz in 450 ml Wasser verrührt und mit Soda auf pH 7,5 gestellt. Nach einer Verweilzeit von 20 Stunden bei 105-107° in einem Tantalautoklaven wird der vorliegende Brei mit heissem Wasser in Lösung gebracht. Das ausgesalzene Produkt wird filtriert, gewaschen und im Vakuum bei 60-70° getrocknet. Man erhält 135,8 g Pulver.

135 g des Nitrodiphenylsulfons werden in 700 ml Wasser, enthaltend 58 g Eisenpulver und 8 ml Eisessig, siedend reduziert. Nach vorsichtiger Zugabe von 9,8 g Soda wird vom Eisenrückstand abfiltriert. Nach Zugabe von Kochsalz wird das ausgefallene Produkt abfiltriert, gewaschen und im Vakuum bei 60-70° getrocknet.

Man erhält in chromatographisch reiner Form 151,2 g der oben formulierten Verbindung.

Beispiel 3

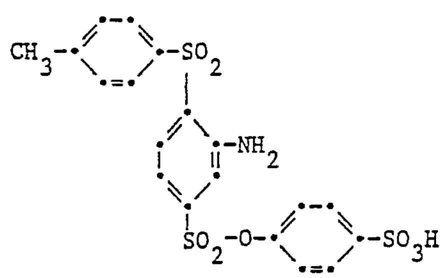

46 g Phenol-4-sulfonsäure (75,6 %ig) werden mit Natronlauge in 300 ml Wasser von Raumtemperatur in Lösung gebracht und auf pH 8,5 eingestellt. Nun lässt man 67,2 g 4-Chlor-3-nitrobenzol-1-sulfochlorid (74 %ig) in 100 ml Aceton gelöst zutropfen und hält mit Natronlauge 10n den pH-Wert bei 8,5. Nach 15 Stunden wird die Ausfällung abgesaugt und gewaschen.

Der Filterkuchen wird zusammen mit 31,6 g Toluol-4-sulfinsäure Na-Salz (95,6 %ig) in 300 ml Wasser versetzt und mit Soda auf pH 8 gestellt. Nach einer Verweilzeit von 20 Stunden bei 104-107° in einem Stahlautoklaven filtriert und wäscht man das erhaltene Produkt. Das feuchte Nutschgut wird in 250 ml Wasser, enthaltend 34 g Eisenpulver und 3 ml Eisessig, eingetragen und bei Siedetemperatur reduziert. Nach vorsichtiger Zugabe von 7 g Soda wird vom Eisenrückstand abfiltriert und das Filtrat zur Trockne eingedampft. Die Ausbeute beträgt 76 g.

Beispiel 4

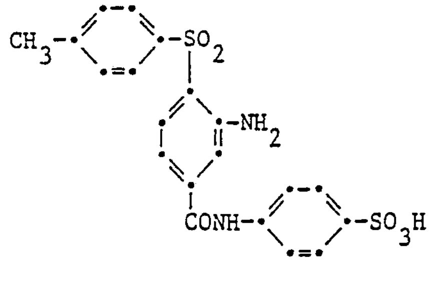

87,5 g Sulfanilsäure (99 %ig) werden unter Rühren in 500 ml Wasser mit Natronlauge konz. auf pH 7-8 gestellt und abgekühlt. Zu der 5° kalten Lösung werden 106,2 g 4-Chlor-3-nitrobenzoylchlorid in 100 ml

Aceton gelöst zugetropft. Man lässt eine Weile bei pH 7 nachrühren und fällt dann das Carbonamid durch Kochsalzzugabe aus. Das filtrierte und gewaschene Produkt ergibt nach dem Trocknen bei 60-70° im Vakuum 163 g. 61,2 g dieser Verbindung werden zusammen mit 30,7 g Toluol-4-sulfinsäure Na-Salz (95,6 %ig) in 300 ml Wasser während einigen Stunden bei Rückflusstemperatur belassen. Das filtrierte und gewaschene Sulfonderivat wird im Vakuum bei 60-70° getrocknet. Man erhält 66,8 g chromatographisch reine Substanz.

66 g des Nitrodiphenylsulfons werden in 250 ml Wasser, enthaltend 30 g Eisenpulver und 2 ml Eisessig, siedend reduziert. Nach vorsichtiger Zugabe von 3,1 g Soda wird vom Eisenrückstand abfiltriert und das reine Produkt im Filtrat durch Kochsalzzugabe ausgefällt. Man filtriert, wäscht und trocknet im Vakuum bei 60-70°. Die Ausbeute der oben formulierten Verbindung beträgt 62 g.

Beispiel 5

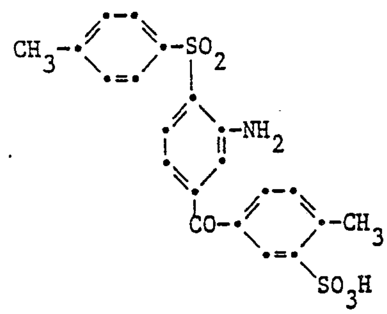

138 g 1-Chlor-2-nitro-4'-methyl-benzophenon werden in 450 ml Oleum 25 %ig bei 20-48° sulfiert. Das auf Eis ausgetragene und mit Kochsalz ausgefällte Produkt wird filtriert und erneut in Wasser gelöst und mit Kaliumchlorid ausgesalzen. Man wäscht den abgesaugten Niederschlag und trocknet im Vakuum bei 60-70°. Die Ausbeute beträgt 221,4 g.

59,8 g dieser Verbindung werden zusammen mit 20,46 g Toluol-4-sulfinsäure Na-Salz (95,6 %ig) in 300 ml Wasser auf pH 8 gestellt und für einige Zeit zum Sieden erwärmt. Das kalt filtrierte und gewaschene Sulfonderivat wird als feuchte Ware in 300 ml Wasser, enthaltend 20 g Eisenpulver und 2 ml Eisessig, eingetragen und siedend zum Amin reduziert. Nach vorsichtiger Zugabe von 3,1 g Soda wird vom Eisenrückstand abgesaugt und das Produkt im Filtrat mit Kochsalz ausgefällt. Filtriert, gewaschen und im Vakuum bei 60-70° getrocknet erhält man 49,7 g der oben formulierten Verbindung.

Beispiel 6

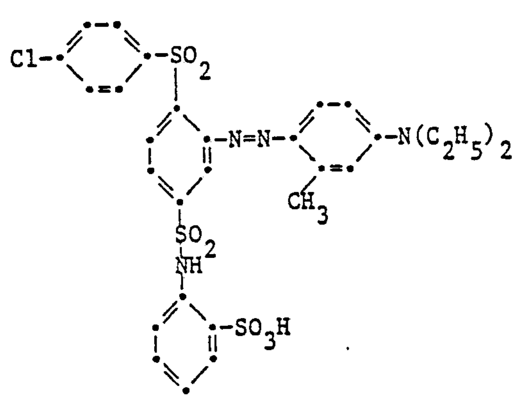

12,53 g der Verbindung aus Beispiel 1 (80,2 %ig) werden in 100 ml Eiswasser neutral verrührt und mit 5 ml Natriumnitrit 4n vermischt. Nach Zugabe von 6 ml Salzsäure konz. lässt man die Suspension einige Zeit nachrühren und zerstört dann einen allfälligen Nitritüberschuss mit Sulfaminsäure.

3,26 g N,N-Diäthyl-m-toluidin werden in 40 ml Wasser, enthaltend 2,12 g Soda, emulgiert. Die oben hergestellte Suspension des Diazoniumsalzes wird bei einem mit Natronlauge konstant gehaltenen pH-Wert von 8,5 zugetropft und das Kupplungsprodukt nach einiger Zeit mit Salzsäure konz. angesäuert. Der filtrierte und gewaschene Farbstoff wird im Vakuum bei 60-70° getrocknet.

Man erhält 10,41 g eines mit Soda coupierten, wasserlöslichen Farbstoffes, der aus schwach saurem Bad gefärbt auf Polyamid brillante rote Töne ergibt. Der Farbstoff verfügt über ein hervorragendes Aufbauvermögen und vorzügliche Lichtechtheit, sehr gute Formaldehydbeständigkeit und gute Nassechtheiten.

Beispiel 7

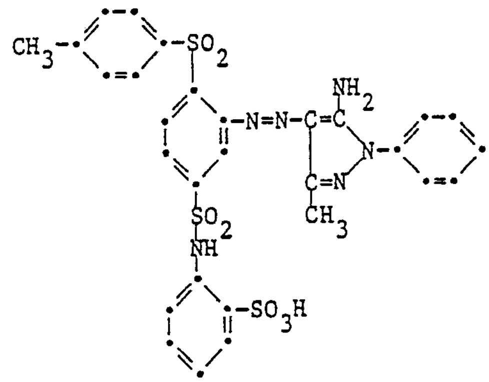

8,87 g 2-Amino-4'-methyl-diphenylsulfon-4-sulfanilid-2"-sulfonsäure (81,5 %ig) werden in 75 ml Eiswasser neutral verrührt und nach dem Mischen mit 3,75 ml Natriumnitrit 4n mit 4,5 ml Salzsäure konz. versetzt. Man lässt die Suspension etwas nachrühren und zerstört einen geringfügigen Nitritüberschuss mit Sulfaminsäure. Danach gibt man die Lösung von 2,7 g 3-Methyl-1-phenyl-5-pyrazolimin (96,3 %ig) in 20 ml Wasser und 2 ml Salzsäure konz. zu und lässt bei Raumtemperatur auskuppeln. Der filtrierte Farbstoff wird in Wasser mit Natronlauge bei pH 8 in Lösung gebracht und mit Kochsalz ausgefällt. Anschliessend wird der filtrierte und gewaschene Farbstoff im Vakuum bei 60-70° getrocknet.

Man erhält 7,51 g Farbstoff, der aus schwach saurem Bad gefärbt, auf Polyamid brillante gelbe Töne ergibt. Der Farbstoff verfügt über ein hervorragendes Aufbauvermögen und gute Allgemeinechtheiten.

Beispiel 8

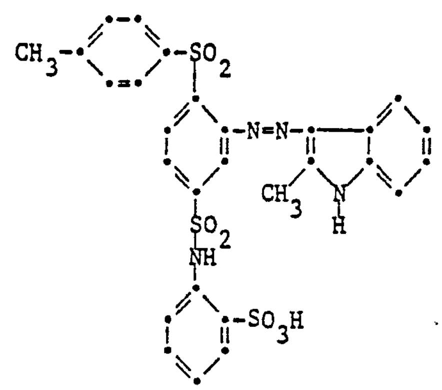

8,87 g 2-Amino-4'-methyl-diphenylsulfon-4-sulfoanilid-2"-sulfonsäure (81,5 %ig) werden wie in Beispiel 7 diazotiert. Nun lässt man eine Lösung von 1,97 g 2-Methylindol in 6 ml Salzsäure konz. zutropfen und führt die Kupplung bei Raumtemperatur zu Ende. Der abgenutschte Farbstoff wird in Wasser mit Natronlauge alkalisch gelöst und mit Kochsalz zur Ausfällung gebracht. Man filtriert, wäscht und trocknet im Vakuum bei 60-70° und erhält 5,53 g Farbstoff, der Polyamidmaterial aus schwach saurem Bad in brillanten, rotstichig gelben Tönen färbt. Das Aufbauvermögen, die Lichtechtheit, die Formaldehyd- und Säurebeständigkeit sind sehr gut.

Beispiel 9

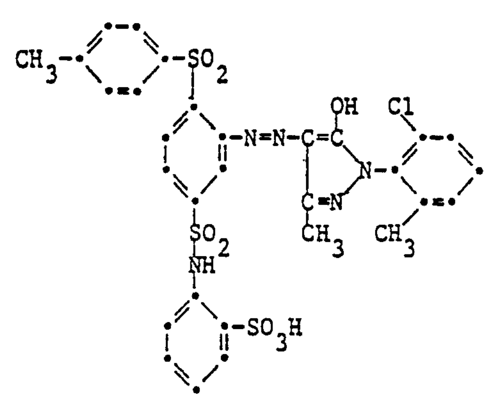

8,87 g 2-Amino-4'-methyl-diphenylsulfon-4-sulfoanilid-2"-sulfonsäure (81,5 %ig) werden wie in Beispiel 7 diazotiert. 6,38 g 1-(2'-Chlor-6'-methylphenyl-3-methylpyrazolon-(5) (52,4 %ig) werden in 50 ml Wasser mit 8 ml Natronlauge 2n auf 70° erwärmt und klärfiltriert. In das auf 2° abgekühlte Filtrat lässt man die oben hergestellte Suspension des Diazoniumsalzes eintropfen und hält mit Natronlauge 2n gleichzeitig den pH-Wert bei ca. 9 konstant. Der mit Kaliumchlorid ausgefällte Farbstoff wird filtriert, gewaschen und im Vakuum bei 60-70° getrocknet.

Man erhält 8,5 g Farbstoff, der Polyamidmaterial aus schwach saurem Bad in brillanten, rotstichig gelben Tönen färbt und über gute Allgemeinechtheiten verfügt.

Beispiel 10

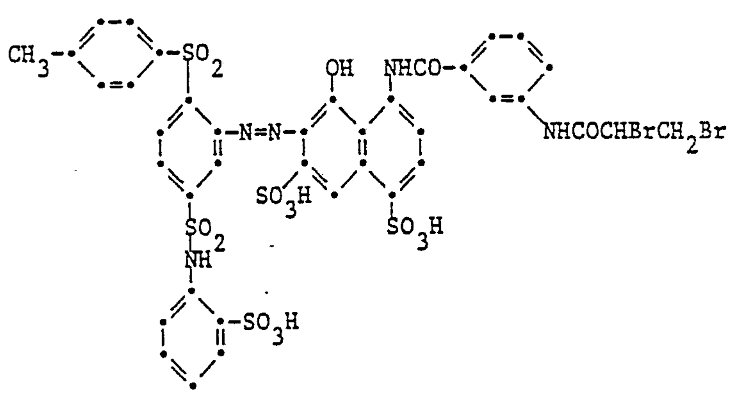

8,87 g 2-Amino-4'-methyl-diphenylsulfon-4-sulfanilid-2"-sulfonsäure (81,5 %ig) werden wie in Beispiel 7 diazotiert. 17,84 g 3-α,β-Dibrompropionylaminobenzoyl-K-säure (55,1 %ig) löst man in 80 ml Wasser bei pH 7 und kühlt ab auf 2°. Man fügt die Diazosuspension tropfenweise hinzu und hält den pH-Wert mit Natronlauge bei 7 konstant. Der Farbstoff wird mit Kaliumchlorid ausgesalzen, abgesaugt und im Vakuum bei 60-70° getrocknet.

Man erhält 14,3 g.

Wenn man wie in den Beispielen 1 bis 10 verfährt, jedoch als Diazokomponente eine Verbindung der Formel

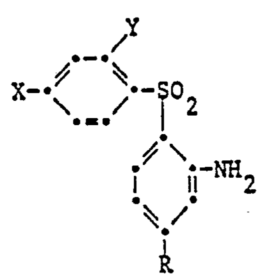

und als Kupplungskomponente die in der folgenden Tabelle aufgeführten Verbindungen einsetzt, so erhält man ebenfalls wertvolle Farbstoffe mit ähnlich guten Eigenschaften, die Polyamid oder Wolle in der angegebenen Nuance anfärben.

(Siehe Tabelle Seite 15 ff.)

Tabelle

| Bei-spiel | Diazokomponente Y | X | R | Kupplungskomponente | Nuance auf Polyamid |
|---|---|---|---|---|---|
| 1 | H | $-CH_3$ | $-SO_2NHCH_2CH_2SO_3H$ | 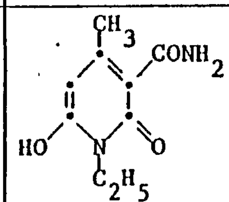 | grünstichig gelb |
| 2 | H | do. | do. | 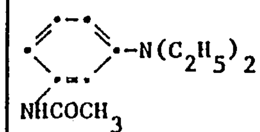 | rot |
| 3 | H | do. | do. | 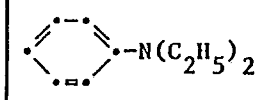 | gelbstichig rot |
| 4 | H | do. | do. | 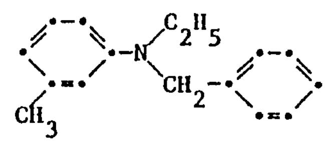 | do. |
| 5 | H | do. | do. | 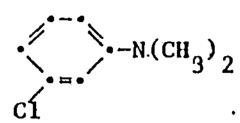 | rotstichig orange |

Tabelle (Fortsetzung)

| Bei-spiel | Diazokomponente Y | X | R | Kupplungskomponente | Nuance auf Polyamid |
|---|---|---|---|---|---|
| 6 | H | $-CH_3$ | $-SO_2NHCH_2CH_2SO_3H$ | 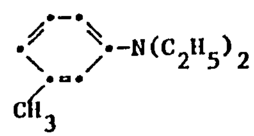 | rot |
| 7 | H | do. | do. | 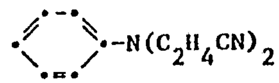 | gelbstichig orange |
| 8 | H | do. | do. | 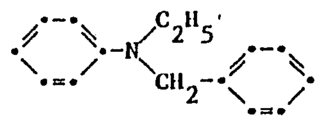 | rotstichig orange |
| 9 | H | do. | do. | 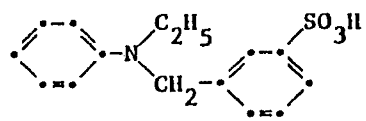 | do. |
| 10 | H | do. | do. | 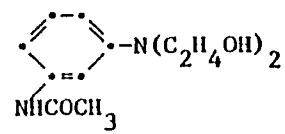 | rot |
| 11 | H | do. | do. | 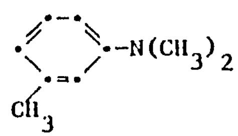 | do. |

Tabelle (Fortsetzung)

| Beispiel | Diazokomponente Y | X | R | Kupplungskomponente | Nuance auf Polyamid |
|---|---|---|---|---|---|
| 12 | H | $-CH_3$ | $-SO_2NHCH_2CH_2SO_3H$ | 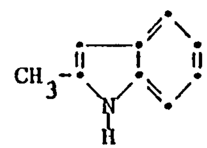 | rotstichig gelb |
| 13 | H | do. | do. | 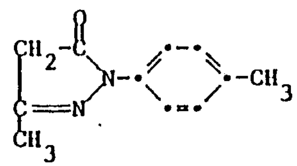 | do. |
| 14 | H | do. | $-SO_2N(CH_3)CH_2CH_2SO_3H$ | 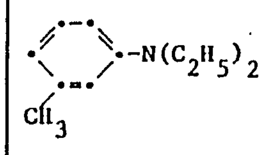 | rot |
| 15 | H | do. | do. | 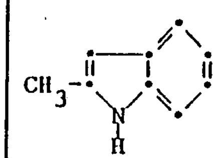 | rotstichig gelb |
| 16 | H | -H | $-SO_2NHCH_2CH_2SO_3H$ | 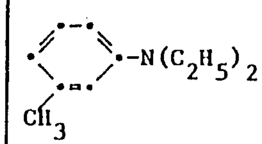 | rot |
| 17 | H | -Cl | do. | do. | do. |

Tabelle (Fortsetzung)

| Beispiel | Diazokomponente Y | X | R | Kupplungskomponente | Nuance auf Polyamid |
|---|---|---|---|---|---|
| 18 | H | $C_2H_5O-$ | $-SO_2NHCH_2CH_2SO_3H$ | 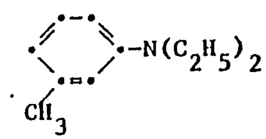 | rot |
| 19 | H | $-NHCOCH_3$ | do. | do. | do. |
| 20 | H | $-CH_3$ | 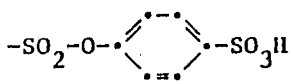 | 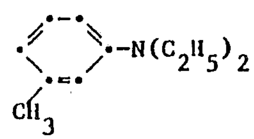 | rot |
| 21 | H | do. | do. | 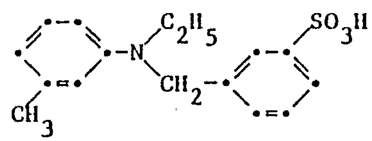 | gelbstichig rot |
| 22 | H | do. | do. | 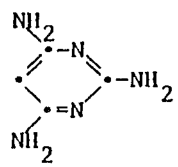 | rotstichig gelb |
| 23 | H | do. | do. | 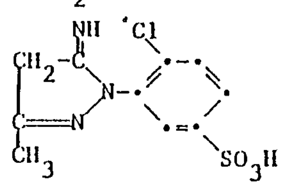 | grünstichig gelb |

Tabelle (Fortsetzung)

| Bei-spiel | Diazokomponente Y | X | R | Kupplungskomponente | Nuance auf Polyamid |
|---|---|---|---|---|---|
| 24 | H | $-NHCOCH_3$ | 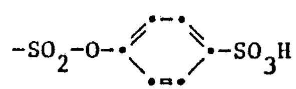 | 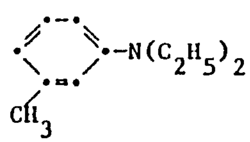 | rot |
| 25 | H | do. | 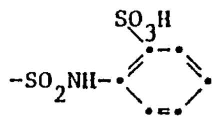 | do. | do. |
| 26 | H | -H | do. | do. | do. |
| 27 | H | $-CH_3$ | do. | do. | do. |
| 28 | H | -Cl | 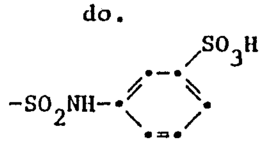 | do. | do. |
| 29 | H | -H | do. | do. | do. |
| 30 | H | $-CH_3$ | do. | do. | do. |
| 31 | H | -Cl | 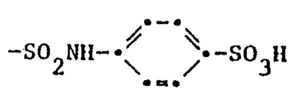 | do. | do. |
| 32 | H | -H | do. | do. | do. |
| 33 | H | $-CH_3$ | do. | do. | do. |

Tabelle (Fortsetzung)

| Bei-spiel | Diazokomponente Y | X | R | Kupplungskomponente | Nuance auf Polyamid |
|---|---|---|---|---|---|
| 34 | H | $-CH_3$ | 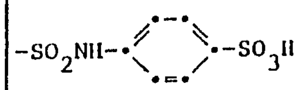 | 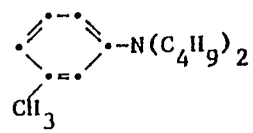 | rot |
| 35 | H | $-CH_3$ | do. | 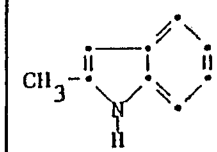 | rotstichig gelb |
| 36 | H | do. | do. | 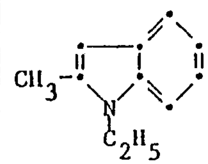 | do. |
| 37 | H | $-NHCOCH_3$ | 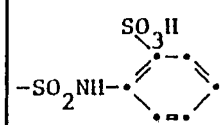 | do. | do. |
| 38 | H | $-CH_3$ | do. | 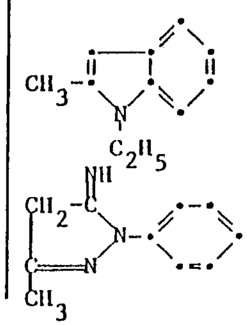 | do. |
| 39 | H | $-NHCOCH_3$ | do. | | gelb |

Tabelle (Fortsetzung)

| Bei-spiel | Diazokomponente | | | Kupplungskomponente | Nuance auf Polyamid |
|---|---|---|---|---|---|
| | Y | X | R | | |
| 40 | H | $-CH_3$ | 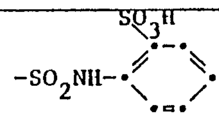 | 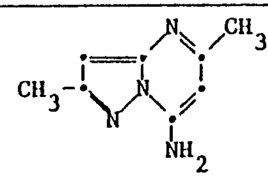 | gelb |
| 41 | H | $-CH_3$ | do. | 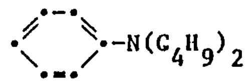 | gelbstichig rot |
| 42 | H | -Cl | 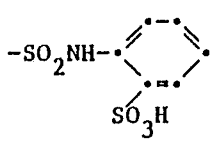 | 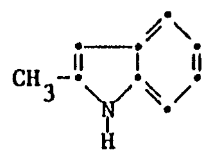 | rotstichig gelb |
| 43 | H | do. | do. | 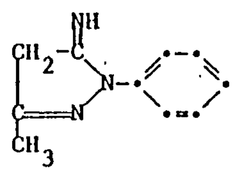 | gelb |
| 44 | H | do. | do. | 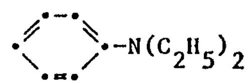 | gelbstichig rot |
| 45 | H | do. | do. | 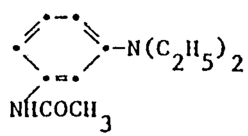 | rot |

Tabelle (Fortsetzung)

| Beispiel | Diazokomponente Y | X | R | Kupplungskomponente | Nuance auf Polyamid |
|---|---|---|---|---|---|
| 46 | H | -Cl | 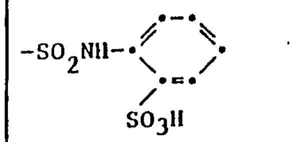 | 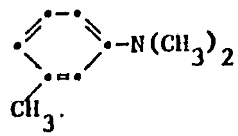 | rot |
| 47 | H | do. | 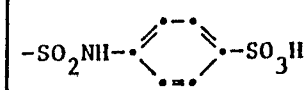 | 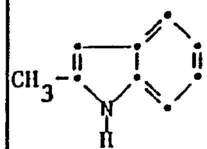 | rotstichig gelb |
| 48 | H | do. | 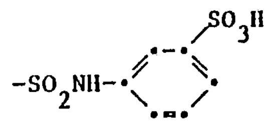 | do. | do. |
| 49 | H | -H | do. | do. | do. |
| 50 | H | do. | 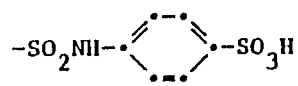 | do. | do. |
| 51 | H | do. | 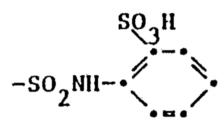 | do. | do. |
| 52 | H | $-CH_3$ | 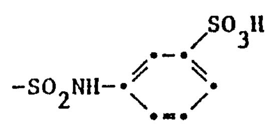 | do. | do. |

Tabelle (Fortsetzung)

| Beispiel | Diazokomponente Y | X | R | Kupplungskomponente | Nuance auf Polyamid |
|---|---|---|---|---|---|
| 53 | H | $-CH_3$ | 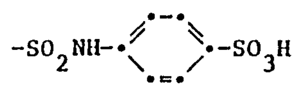 | 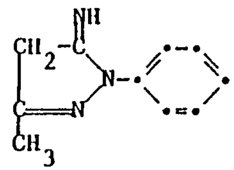 | gelb |
| 54 | H | Cl | 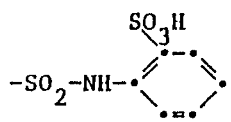 | 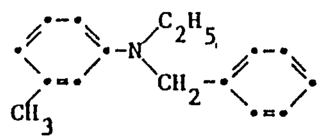 | gelbstichig rot |
| 55 | H | do. | do. | 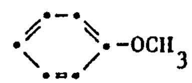 * | gelbstichig orange |
| 56 | H | do. | do. | 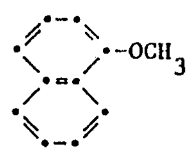 * | orange |
| 57 | H | do. | do. | 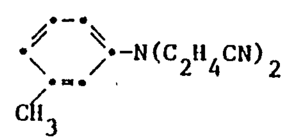 | rotstichig orange |
| 58 | H | do. | do. | 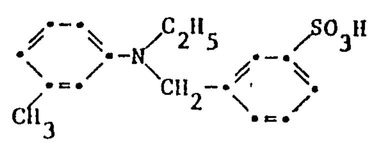 | gelbstichig rot |

*) In diesen Fällen (Beispiele 55 und 56) wurde nicht mit den dort angegebenen Kupplungskomponenten, sondern mit den entsprechenden unverätherten Hydroxyverbindungen, d. h. mit Phenol bzw. Naphthol, gekuppelt, und nach der Kupplung wurden die Hydroxygruppen alkyliert. Dass in den Beispielen 55 und 56 die bereits verätherten Hydroxyverbindungen als Kupplungskomponenten genannt sind, dient lediglich dazu, die Struktur der Endprodukte klarzustellen.

Tabelle (Fortsetzung)

| Bei-spiel | Diazokomponente Y | X | R | Kupplungskomponente | Nuance auf Polyamid |
|---|---|---|---|---|---|
| 59 | H | 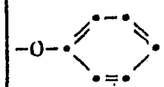 | 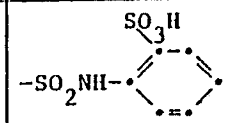 | 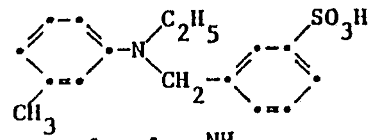 | gelbstichig rot |
| 60 | H | -Cl | do. | 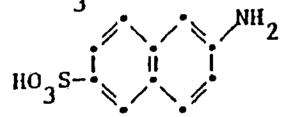 | rot |
| 61 | H | do. | do. | 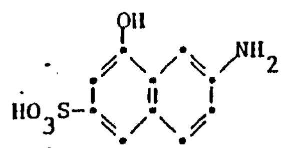 | violett |
| 62 | H | do. | do. | 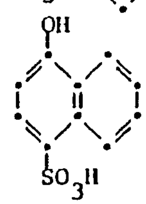 | braun |
| 63 | H | $-CH_3$ | 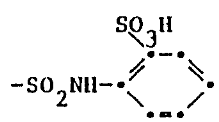 | 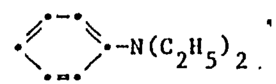 | gelbstichig rot |

Tabelle (Fortsetzung)

| Beispiel | Diazokomponente Y | X | R | Kupplungskomponente | Nuance auf Polyamid |
|---|---|---|---|---|---|
| 64 | H | $-CH_3$ | 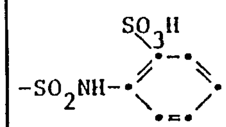 | 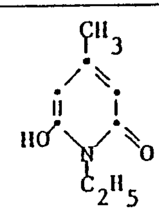 | grünstichig gelb |
| 65 | H | do. | do. | 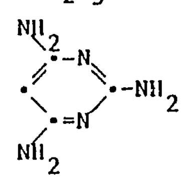 | rotstichig gelb |
| 66 | H | do. | do. | 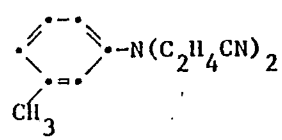 | rotstichig orange |
| 67 | H | do. | 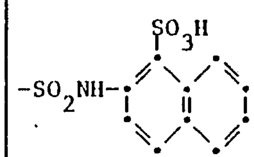 | 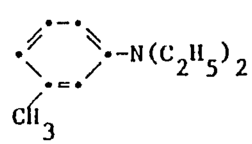 | rot |
| 68 | H | do. | do. | 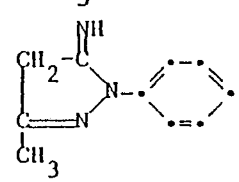 | gelb |

Tabelle (Fortsetzung)

| Beispiel | Diazokomponente Y | X | R | Kupplungskomponente | Nuance auf Polyamid |
|---|---|---|---|---|---|
| 69 | H | $-CH_3$ | 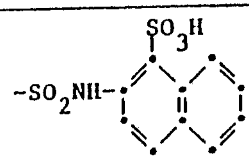 | 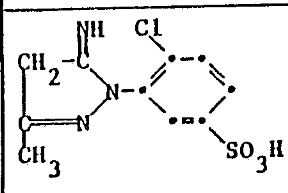 | gelb |
| 70 | H | do. | do. | 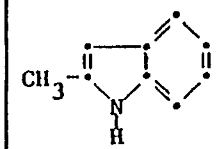 | rotstichig gelb |
| 71 | H | -H | do. | 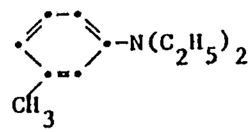 | rot |
| 72 | H | -Cl | do. | do. | do. |
| 73 | H | do. | do. | 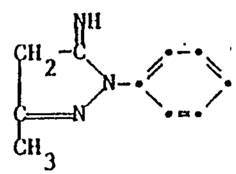 | gelb |
| 74 | H | -H | do. | do. | do. |
| 75 | H | do. | do. | 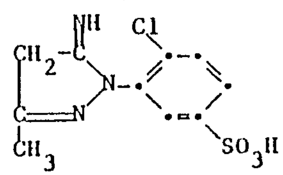 | do. |

Tabelle (Fortsetzung)

Tabelle

| Bei-spiel | Diazokomponente Y | X | R | Kupplungskomponente | Nuance auf Polyamid |
|---|---|---|---|---|---|
| 76 | H | $-CH_3$ | 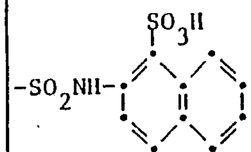 | 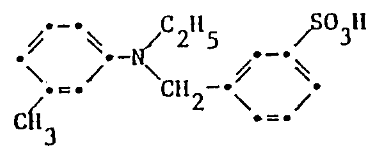 | gelbstichig rot |
| 77 | H | do. | 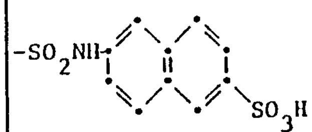 | 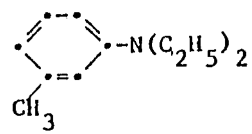 | rot |
| 78 | H | do. | do. | 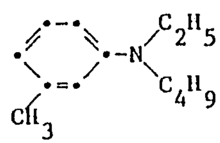 | do. |
| 79 | H | do. | do. | 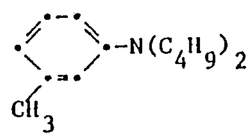 | do. |
| 80 | H | -H | do. | do. | do. |
| 81 | H | $-CH_3$ | 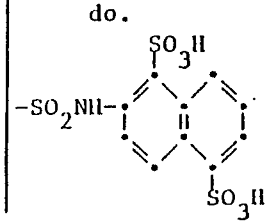 | 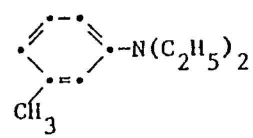 | rot |

Tabelle (Fortsetzung)

| Beispiel | Diazokomponente Y | X | R | Kupplungskomponente | Nuance auf Polyamid |
|---|---|---|---|---|---|
| 82 | H | $-CH_3$ | 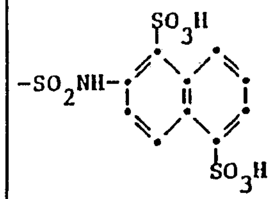 | 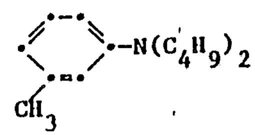 | rot |
| 83 | H | do. | do. | 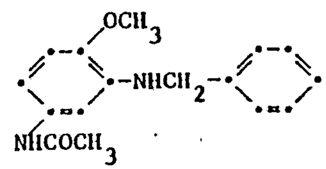 | blaustichig rot |
| 84 | H | do. | do. | 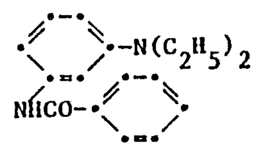 | rot |
| 85 | H | do. | do. | 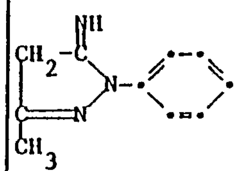 | gelb |
| 86 | H | do. | do. | 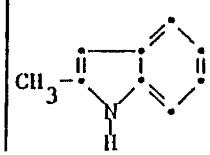 | rotstichig gelb |

Tabelle (Fortsetzung)

| Bei-spiel | Diazokomponente Y | X | R | Kupplungskomponente | Nuance auf Polyamid |
|---|---|---|---|---|---|
| 87 | H | $-CH_3$ | 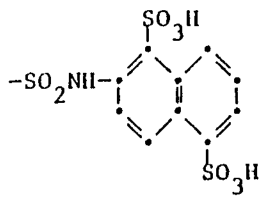 | 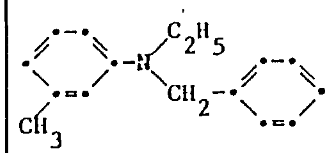 | gelbstichig rot |
| 88 | H | -Cl | do. | 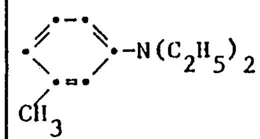 | rot |
| 89 | H | -H | do. | do. | do. |
| 90 | H | do. | do. | 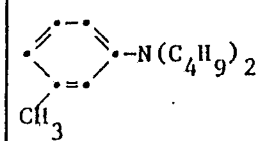 | do. |
| 91 | H | -Cl | do. | do. | do. |
| 92 | H | do. | 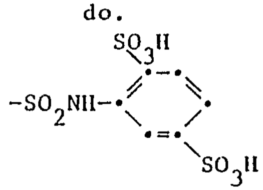 | do. | do. |
| 93 | H | do. | do. | 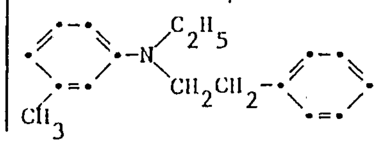 | do. |

Tabelle (Fortsetzung)

| Beispiel | Diazokomponente Y | X | R | Kupplungskomponente | Nuance auf Polyamid |
|---|---|---|---|---|---|
| 94 | H | -H | 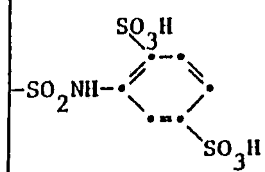 | 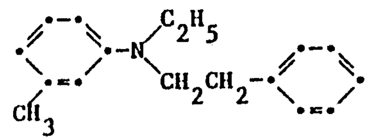 | rot |
| 95 | H | -Cl | do. | 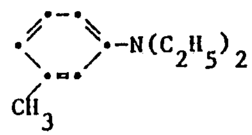 | do. |
| 96 | H | -Cl | 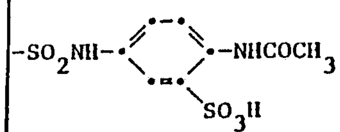 | do. | do. |
| 97 | H | $-CH_3$ | do. | do. | do. |
| 98 | H | do. | 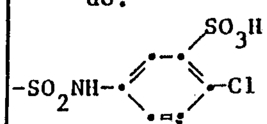 | do. | do. |
| 99 | H | -Cl | do. | do. | do. |
| 100 | H | do. | 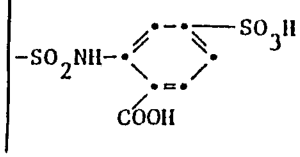 | do. | do. |

Tabelle (Fortsetzung)

| Beispiel | Diazokomponente Y | X | R | Kupplungskomponente | Nuance auf Polyamid |
|---|---|---|---|---|---|
| 101 | H | $-CH_3$ | 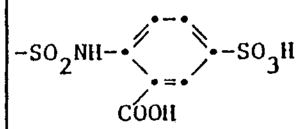 | 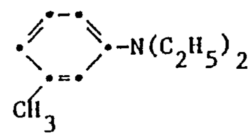 | rot |
| 102 | H | do. | 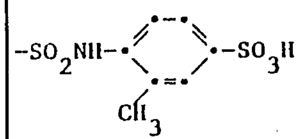 | do. | do. |
| 103 | H | -H | do. | do. | do. |
| 104 | H | do. | 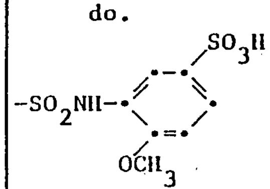 | do. | do. |
| 105 | H | $-CH_3$ | do. | do. | do. |
| 106 | H | -Cl | 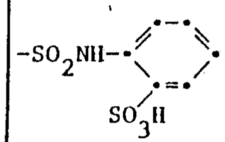 | 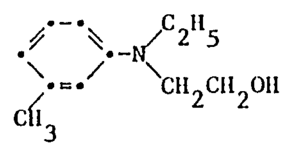 | do. |
| 107 | H | do. | do. | 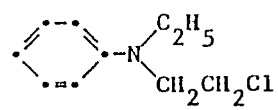 | gelbstichig rot |

Tabelle (Fortsetzung)

| Bei- spiel | Diazokomponente Y | X | R | Kupplungskomponente | Nuance auf Polyamid |
|---|---|---|---|---|---|
| 108 | H | $-CH_3$ | 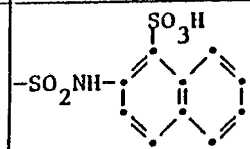 | 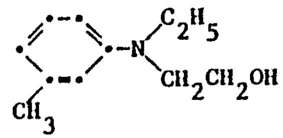 | rot |
| 109 | H | $-CH_3$ | $-SO_2NHCH_2CH_2SO_3H$ | 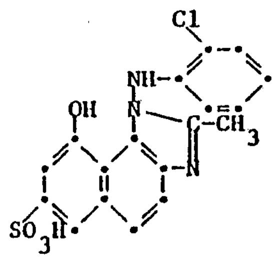 | do. |
| 110 | H | do. | do. | 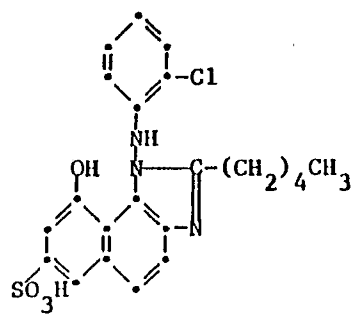 | do. |
| 111 | H | do. | 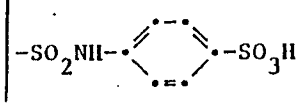 | do. | do. |

Tabelle (Fortsetzung)

| Beispiel | Diazokomponente Y | X | R | Kupplungskomponente | Nuance auf Polyamid |
|---|---|---|---|---|---|
| 112 | $-OCH_3$ | $-CH_3$ | 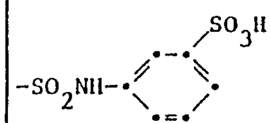 | 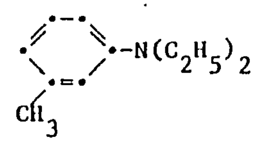 | rot |
| 113 | $-OCH_3$ | -H | do. | do. | do. |
| 114 | H | $-CH_3$ | 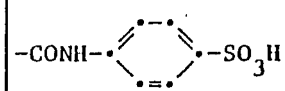 | 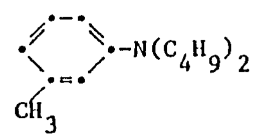 | gelbstichig rot |
| 115 | H | do. | do. | 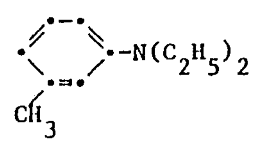 | do. |
| 116 | H | do. | do. | 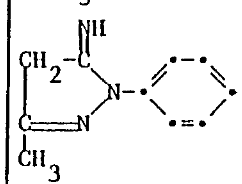 | gelb |
| 117 | H | do. | do. | 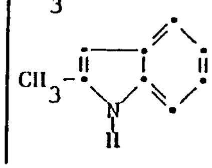 | rotstichig gelb |

Tabelle (Fortsetzung)

| Beispiel | Diazokomponente Y | X | R | Kupplungskomponente | Nuance auf Polyamid |
|---|---|---|---|---|---|
| 118 | H | $-CH_3$ | 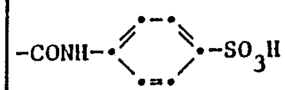 | 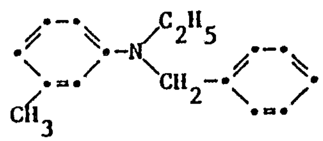 | rotstichig orange |
| 119 | H | do. | 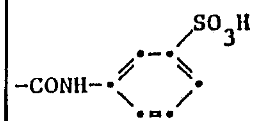 | 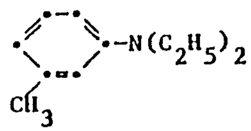 | gelbstichig rot |
| 120 | H | -H | do. | do. | do. |
| 121 | H | -Cl | do. | do. | do. |
| 122 | H | $-CH_3$ | do. | 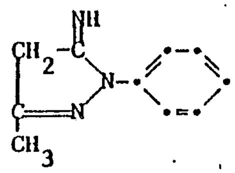 | gelb |
| 123 | H | -H | do. | do. | do. |
| 124 | H | -Cl | do. | do. | do. |
| 125 | H | $-CH_3$ | do. | 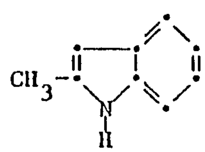 | rotstichig gelb |

Tabelle (Fortsetzung)

| Bei-spiel | Diazokomponente Y | X | R | Kupplungskomponente | Nuance auf Polyamid |
|---|---|---|---|---|---|
| 126 | H | -H | 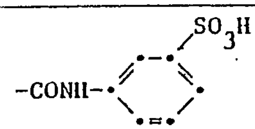 | 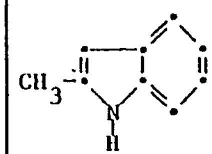 | rotstichig gelb |
| 127 | H | -Cl | do. | do. | do. |
| 128 | H | $-CH_3$ | 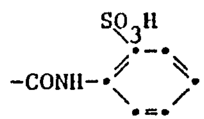 | 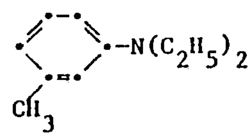 | gelbstichig rot |
| 129 | H | -H | do. | do. | do. |
| 130 | H | -Cl | do. | do. | do. |
| 131 | H | $-CH_3$ | do. | 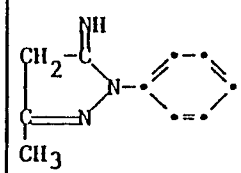 | gelb |
| 132 | H | -H | do. | do. | do. |
| 133 | H | -Cl | do. | do. | do. |
| 134 | H | $-CH_3$ | do. | 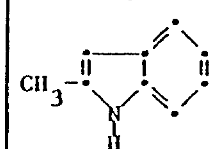 | rotstichig gelb |
| 135 | H | -H | do. | do. | do. |
| 136 | H | -Cl | do. | do. | do. |

Tabelle (Fortsetzung)

| Beispiel | Diazokomponente Y | X | R | Kupplungskomponente | Nuance auf Polyamid |
|---|---|---|---|---|---|
| 137 | H | $-CH_3$ | $-CONHCH_2CH_2SO_3H$ | 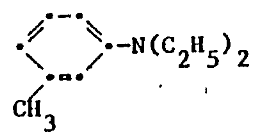 | gelbstichig rot |
| 138 | H | do. | do. | 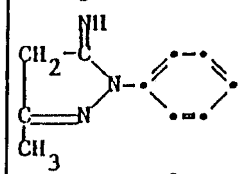 | gelb |
| 139 | H | do. | do. | 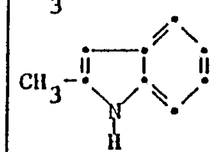 | rotstichig gelb |
| 140 | H | do. | do. | 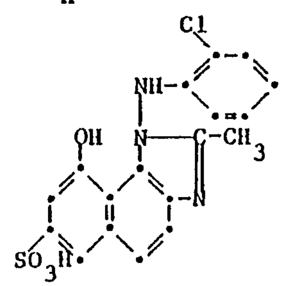 | rot |
| 141 | H | do. | -CONH- 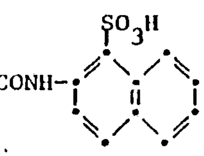 | 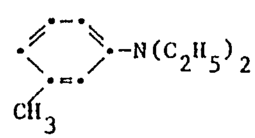 | gelbstichig rot |

Tabelle (Fortsetzung)

| Bei-spiel | Diazokomponente | | | Kupplungskomponente | Nuance auf Polyamid |
|---|---|---|---|---|---|
| | Y | X | R | | |
| 142 | H | $-CH_3$ | 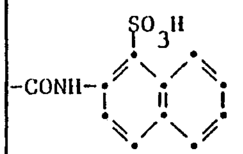 | 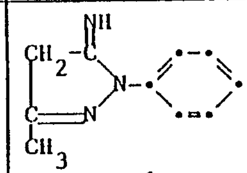 | gelb |
| 143 | H | do. | do. | 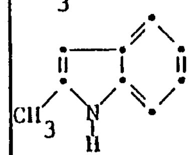 | rotstichig gelb |
| 144 | H | do. | 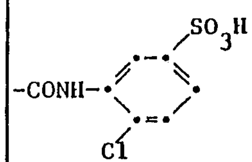 | do. | do. |
| 145 | H | do. | do. | 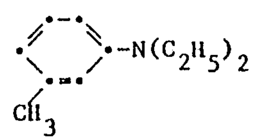 | gelbstichig rot |
| 146 | H | do. | do. | 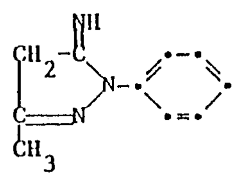 | gelb |
| 147 | H | -H | do. | do. | do. |
| 148 | H | -Cl | do. | do. | do. |

Tabelle (Fortsetzung)

| Beispiel | Diazokomponente Y | X | R | Kupplungskomponente | Nuance auf Polyamid |
|---|---|---|---|---|---|
| 149 | H | -Cl | 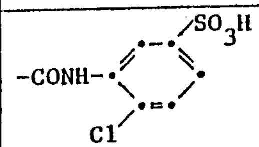 | 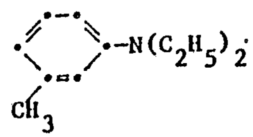 | gelbstichig rot |
| 150 | H | -H | do. | do. | do. |
| 151 | H | $-CH_3$ | 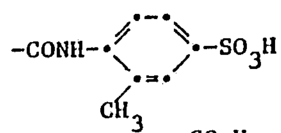 | do. | do. |
| 152 | H | do. | 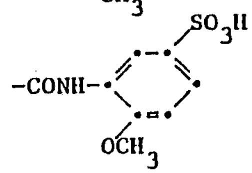 | do. | do. |
| 153 | H | do. | 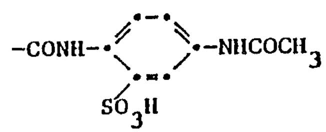 | do. | do. |
| 154 | H | $-CH_3$ | 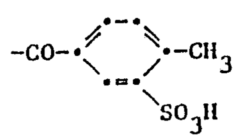 | 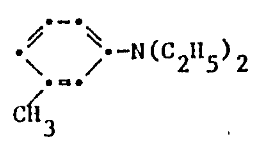 | gelbstichig rot |
| 155 | H | do. | do. | 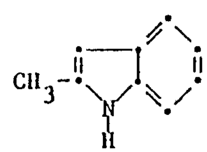 | rotstichig gelb |

Tabelle (Fortsetzung)

| Bei-spiel | Diazokomponente Y | X | R | Kupplungskomponente | Nuance auf Polyamid |
|---|---|---|---|---|---|
| 156 | H | $-CH_3$ | 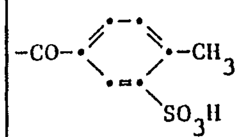 | 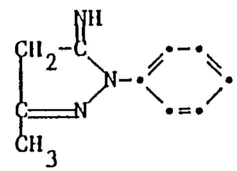 | gelb |
| 157 | H | -H | do. | 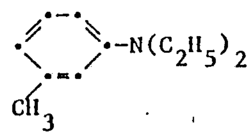 | gelbstichig rot |
| 158 | H | -Cl | do. | do. | do. |
| 159 | H | -H | do. | 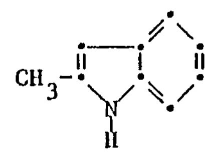 | rotstichig gelb |
| 160 | H | -Cl | do. | do. | do. |
| 161 | H | -H | do. | 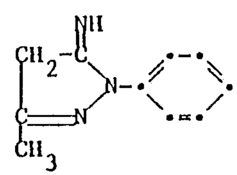 | gelb |
| 162 | H | -Cl | do. | do. | do. |

Tabelle (Fortsetzung)

| Beispiel | Diazokomponente Y | X | R | Kupplungskomponente | Nuance auf Polyamid |
|---|---|---|---|---|---|
| 163 | H | $-CH_3$ | 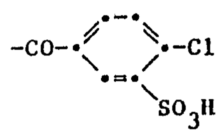 | 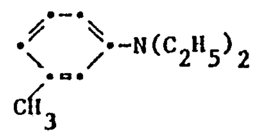 | gelbstichig rot |
| 164 | H | -H | do. | do. | do. |
| 165 | H | -Cl | do. | do. | do. |
| | | | | | Nuance auf Wolle |
| 166 | H | $-CH_3$ | 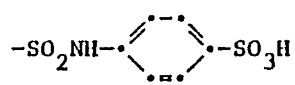 | 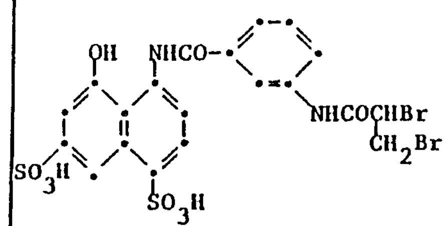 | rot |
| 167 | H | -Cl | do. | do. | do. |
| 168 | H | $-CH_3$ | 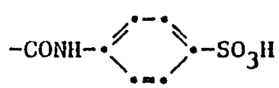 | do. | do. |
| 169 | H | do. | $-SO_2NHCH_2CH_2SO_3H$ | do. | do. |
| 170 | H | do. | 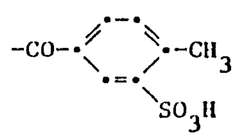 | do. | do. |

Tabelle (Fortsetzung)

| Bei-spiel | Diazokomponente | | | Kupplungskomponente | Nuance auf Wolle |
|---|---|---|---|---|---|
| | Y | X | R | | |
| 171 | H | $-CH_3$ | 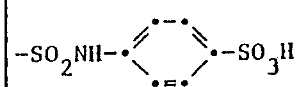 | 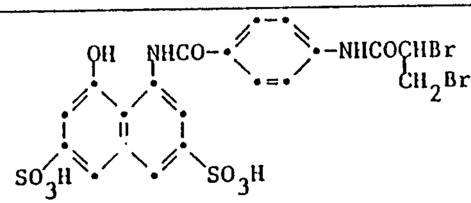 | rot |
| 172 | H | do. | do. | 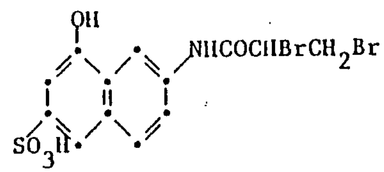 | gelbstichig rot |
| 173 | H | do. | do. | 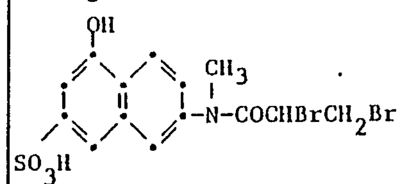 | rotstichig orange |
| 174 | H | do. | do. | 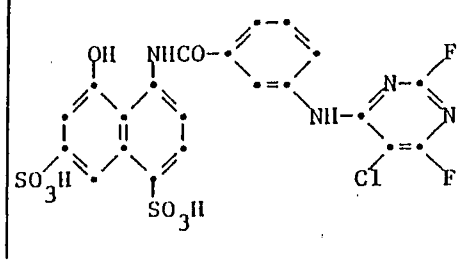 | rot |

Tabelle (Fortsetzung)

| Beispiel | Diazokomponente Y | X | R | Kupplungskomponente | Nuance auf Polyamid |
|---|---|---|---|---|---|
| 175 | H | $CH_3$ | 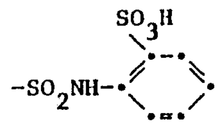 | 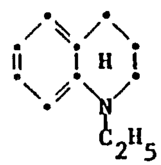 | rot |
| 176 | H | H | dito | dito | rot |
| 177 | H | Cl | dito | dito | rot |
| 178 | H | $CH_3$ | 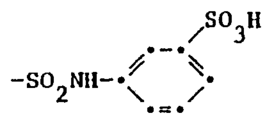 | dito | rot |
| 179 | H | H | dito | dito | rot |
| 180 | H | Cl | dito | dito | rot |
| 181 | H | $CH_3$ | 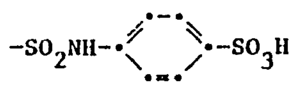 | dito | rot |
| 182 | H | H | dito | dito | rot |
| 183 | H | Cl | dito | dito | rot |
| 184 | H | Cl | 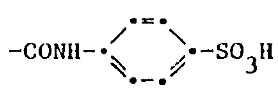 | dito | rot |

Tabelle (Fortsetzung)

| Bei-spiel | Diazokomponente Y | X | R | Kupplungskomponente | Nuance auf Polyamid |
|---|---|---|---|---|---|
| 185 | H | Cl | $-SO_2NHCH_2CH_2SO_3H$ | 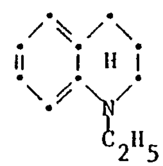 | rot |
| 186 | H | Cl | 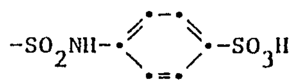 | 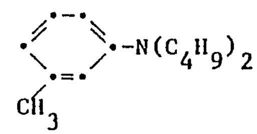 | rot |
| 187 | H | H | dito | dito | rot |
| 188 | H | $CH_3$ | 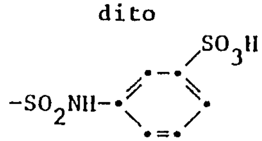 | dito | rot |
| 189 | H | H | dito | dito | rot |
| 190 | H | Cl | dito | dito | rot |
| 191 | H | Cl | $-SO_2NHCH_2CH_2SO_3H$ | dito | rot |
| 192 | H | Cl | 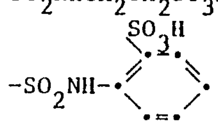 | 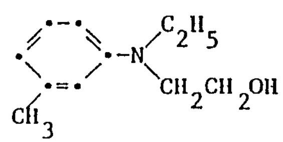 | rot |
| 193 | H | $CH_3$ | dito | dito | rot |
| 194 | H | H | dito | dito | rot |

Tabelle (Fortsetzung)

| Beispiel | Diazokomponente Y | X | R | Kupplungskomponente | Nuance auf Polyamid |
|---|---|---|---|---|---|
| 195 | H | Cl | 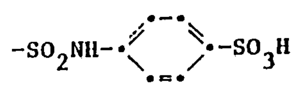 | 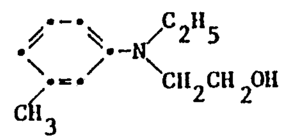 | rot |
| 196 | H | Cl | dito | 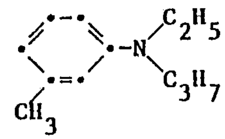 | rot |
| 197 | H | $CH_3$ | dito | dito | rot |
| 198 | H | H | dito | dito | rot |
| 199 | H | $CH_3$ | 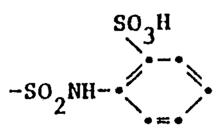 | dito | rot |
| 200 | H | H | dito | dito | rot |
| 201 | H | Cl | dito | dito | rot |
| 202 | H | H | $-SO_2NHCH_2CH_2SO_3H$ | dito | rot |
| 203 | H | $CH_3$ | 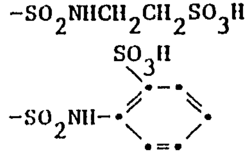 | 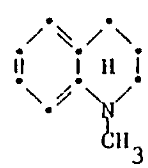 | rot |

Tabelle (Fortsetzung)

| Beispiel | Diazokomponente Y | X | R | Kupplungskomponente | Nuance auf Polyamid |
|---|---|---|---|---|---|
| 204 | Cl | Cl | 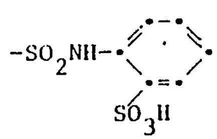 | 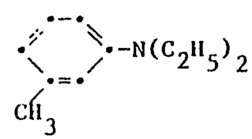 | rot |
| 205 | Cl | Cl | 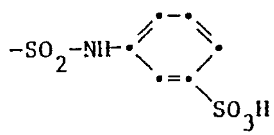 | dito | rot |
| 206 | Cl | Cl | 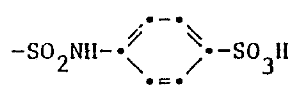 | dito | rot |
| 207<br>207 | Cl | Cl | 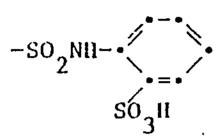 | 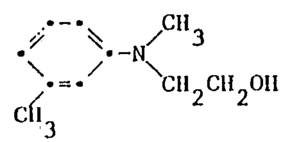 | rot |
| 208 | Cl | Cl | 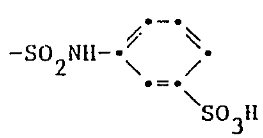 | dito | rot |
| 209 | Cl | Cl | 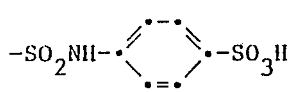 | dito | rot |

Tabelle (Fortsetzung)

| Bei-spiel | Diazokomponente Y | X | R | Kupplungskomponente | Nuance auf Polyamid |
|---|---|---|---|---|---|
| 210 | Cl | Cl | 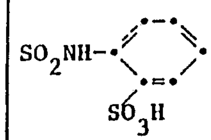 | 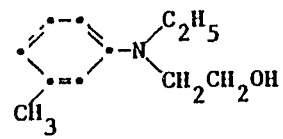 | rot |
| 211 | Cl | Cl | 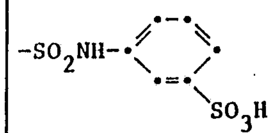 | dito | rot |
| 212 | Cl | Cl | 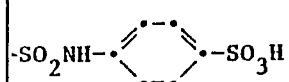 | dito | rot |
| 213 | Cl | Cl | 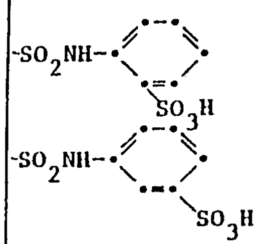 | 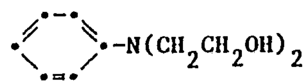 | rot |
| 214 | Cl | Cl | | dito | rot |
| 215 | Cl | Cl | 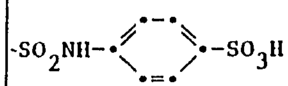 | dito | rot |
| 216 | Cl | Cl | 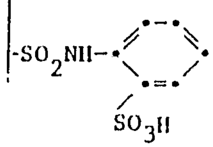 | 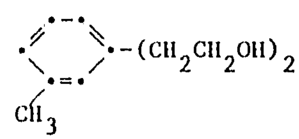 | rot |

Tabelle (Fortsetzung)

| Bei-spiel | Diazokomponente Y | X | R | Kupplungskomponente | Nuance auf Polyamid |
|---|---|---|---|---|---|
| 217 | Cl | Cl | 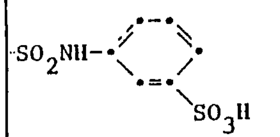 | 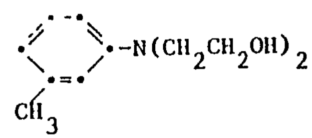 | rot |
| 218 | Cl | Cl | 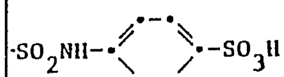 | dito | rot |
| 219 | Cl | Cl | 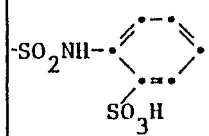 | 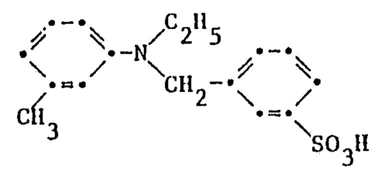 | rot |
| 220 | Cl | Cl | 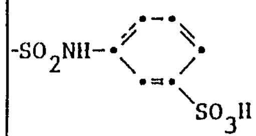 | dito | rot |
| 221 | Cl | Cl | 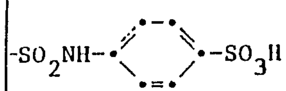 | dito | rot |

Färbevorschrift I

Man färbt 10 Teile Helanca®-Trikot in 500 Teilen einer wässrigen Flotte, die 1 g/l Mononatriumphosphat enthält und mit Dinatriumphosphat auf pH 6 gestellt wird. Der Anteil des Farbstoffes gemäss Beispiel 6 beträgt 1,4 %, bezogen auf das Fasergewicht. Die Färbedauer bei einer Temperatur von 98° beträgt 30 bis 90 Minuten. Das gefärbte Helanca®-Stück wird anschliessend herausgenommen und wie üblich gewaschen und getrocknet.

Man erhält ein tief rot gefärbtes Helanca®-Stück, das eine reine Nuance und sehr gute Gesamtechtheiten aufweist und insbesondere gut formaldehydbeständig ist.

Färbevorschrift II

Man färbt 10 Teile eines Wollstückes in 500 Teilen einer wässrigen Flotte, enthaltend, bezogen auf das Fasergewicht, 4 Gewichtsprozent Ammonsulfat, 1,5 Gewichtsprozent 80 %ige Essigsäure, 1 Gewichtsprozent eines substituierten Alkylaminopolyglykoläthers und 3 Gewichtsprozent des Farbstoffes gemäss Beispiel 10, bei einer Temperatur von 98° während 30 bis 90 Minuten. Anschliessend kühlt man auf 80° ab, neutralisiert durch Zugabe von 2,5 Gewichtsprozent 25 %igem wässrigem Ammoniak und stellt während 15 Minuten bei dieser Temperatur fertig. Das gefärbte Wollstück wird anschliessend herausgenommen und wie üblich gewaschen und getrocknet.

Man erhält ein Wollstück, das eine reine Nuance und gute Gesamtechtheiten aufweist.

**Patentansprüche**

1. Monoazofarbstoffe der Formel

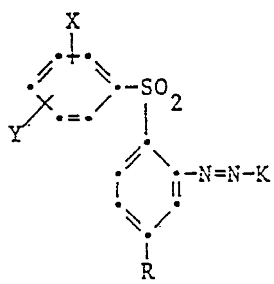

(1),

worin K der Rest einer Kupplungskomponente der Benzol- oder Naphthalinreihe oder der heterocyclischen Reihe und R eine

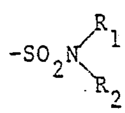 , 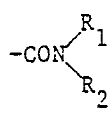 ,

—CO—$R_2$ oder —$SO_2$—O—$R_2$-Gruppe ist, wobei $R_1$ Wasserstoff oder $C_{1-4}$-Alkyl und $R_2$ einen gegebenenfalls substituierten Monosulfo-$C_{1-12}$-Alkylrest, einen Rest der Formel

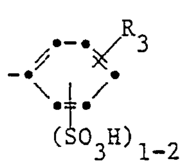

(2)

oder einen Rest der Formel

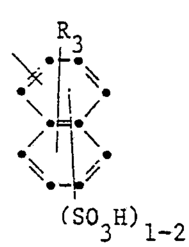

(3),

worin $R_3$ in den Formeln (2) und (3) Wasserstoff, $C_{1-4}$-Alkyl, $C_{1-4}$-Alkoxy, Halogen, $C_{2-6}$-Alkanoylamino oder Carboxy ist, bedeuten und worin X und Y unabhängig voneinander Wasserstoff, $C_{1-4}$-Alkyl, $C_{1-4}$-

Alkoxy, Halogen, $C_{2-6}$-Alkanoylamino, $C_{1-5}$-Alkylsulfonylamino oder einen gegebenenfalls substituierten Phenyl oder Phenoxyrest bedeuten.

2. Monoazofarbstoffe gemäss Anspruch 1, worin K der Rest eines Amino- oder Alkoxybenzols, Amino- oder Alkoxynaphthalins, Naphthols, Aminonaphthols, Pyrazolons, Aminopyrazols, Pyridons, Pyrimidines, Indols, Naphthimidazols, Diphenylamins, Pyrazolo[2,3-a]pyrimidins, Tetrahydrochinolins oder Acetessigsäureamids ist, wobei die oben genannten Reste weitersubstituiert sein können, und R, $R_1$, $R_2$, X und Y die in Anspruch 1 angegebenen Bedeutungen haben.

3. Monoazofarbstoffe gemäss Anspruch 1, worin $R_2$ einen unsubstituierten Monosulfo-$C_{1-12}$-alkylrest oder einen Rest der Formel (2) oder (3) bedeutet, und K, $R_1$, $R_3$, X und Y die in Anspruch 1 angegebenen Bedeutungen haben.

4. Monoazofarbstoffe gemäss Anspruch 3, worin Y Wasserstoff, Chlor oder Methoxy ist, und K, R, $R_1$, $R_2$, $R_3$ und X die in Anspruch 3 angegebenen Bedeutungen haben.

5. Monoazofarbstoffe gemäss Anspruch 4, worin X in p-Stellung zur —$SO_2$-Gruppe an den Phenylring gebunden ist, und K, R, $R_1$, $R_2$, $R_3$ und Y die in Anspruch 4 angegebenen Bedeutungen haben.

6. Monoazofarbstoffe gemäss Anspruch 5, worin K ein N-$C_{1-4}$-Alkylamino- oder N,N-di-$C_{1-4}$-Alkylaminobenzolrest, der im Benzolring durch Methyl, Chlor, Acetylamino und Benzoylamino substituiert sein kann, und worin die N-Alkylreste unabhängig voneinander durch Hydroxy, Chlor, Cyano, Phenyl oder Sulfophenyl substituiert sein können, ein Methoxybenzolrest, ein Methoxynaphthalinrest, ein Aminonaphthalinrest, der durch Sulfo substituiert sein kann, ein Hydroxynaphthalinrest, der durch Sulfo substituiert sein kann, ein Aminonaphtholrest, der im Naphthalinkern durch Sulfo substituiert sein kann, und worin die Aminogruppe durch Methyl, α,β-Dibrompropionyl, α,β-Dibrompropionylaminobenzoyl und 2,6-Difluor-5-chlorpyrimidylaminobenzoyl substituiert sein kann, ein 1-Phenyl-3-methylpyrazolonrest, der im Phenylrest durch Chlor und Methyl substituiert sein kann, ein 1-Phenyl-3-methyl-aminopyrazolrest, der im Phenylrest durch Chlor und Sulfo substituiert sein kann, ein 1-Aethyl-4-methyl-6-hydroxy-pyridon-(2)-rest, der in 3-Stellung durch Carbamoyl substituiert sein kann, ein 2,4,6-Triaminopyrimidinrest, ein 2-Methylindol- oder 1-Aethyl-2-methylindolrest, ein 1-Chlorphenylamino-2-methyl- oder 2-pentyl-naphthimidazolrest, der im Naphthalinkern durch Hydroxy und Sulfo substituiert ist, ein N-Methyl- oder N-Aethyl-1,2,3,4-tetrahydrochinolinrest, oder ein 2,5-Dimethyl-7-amino-pyrazolo[2,3-a]pyrimidinrest und R eine

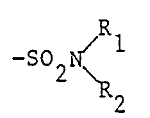 , 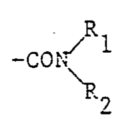 ,

—CO—$R_2$ oder —$SO_2$—O—$R_2$-Gruppe ist, wobei $R_1$ Wasserstoff oder Methyl und $R_2$ β-Sulfoäthyl, 2-, 3- oder 4-Sulfophenyl, wobei der Phenylring durch Methyl, Methoxy, Chlor, Acetylamino und Carboxy substituiert sein kann, 2,5-Disulfophenyl oder Monosulfo- oder Disulfonaphthyl ist, und worin X Wasserstoff, Methyl, Chlor, Aethoxy, Acetylamino oder Phenoxy und Y Wasserstoff, Chlor oder Methoxy ist.

7. Monoazofarbstoffe gemäss Anspruch 6, worin K ein N,N-Di-$C_{1-4}$-Alkylaminobenzolrest, der im Benzolkern durch Methyl, Chlor, Acetylamino und Benzoylamino substituiert sein kann, und worin die N-Alkylreste unabhängig voneinander durch Hydroxy, Chlor, Cyano, Phenyl oder Sulfophenyl substituiert sein können, ein 1-Phenyl-3-methyl-aminopyrazolrest, der im Phenylrest durch Chlor und Sulfo substituiert sein kann, oder ein 2-Methylindol- oder 1-Aethyl-2-methylindolrest ist und R, X und Y die in Anspruch 6 angegebenen Bedeutungen haben.

8. Monoazofarbstoffe gemäss Anspruch 7, worin K ein N,N-Diäthyl-3-methylanilinrest, ein N-Aethyl-N-β-hydroxyäthyl-3-methylanilinrest, ein 1-Phenyl-3-methyl-5-aminopyrazolrest oder ein 2-Methylindolrest R eine β-Sulfoäthylaminosulfonyl-, o-Sulfophenylaminosulfonyl-, p-Sulfophenylaminosulfonyl-, p-Sulfophenylaminocarbonyl- oder p-Methyl-m-sulfobenzoylrest, X Methyl oder Chlor und Y Wasserstoff ist.

9. Verfahren zur Herstellung von Monoazofarbstoffen der Formel

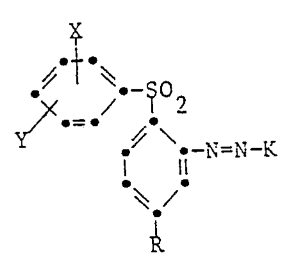

(1),

worin K, R, X und Y die in Anspruch 1 angegebenen Bedeutungen haben, dadurch gekennzeichnet, dass man eine Diazokomponente der Formel

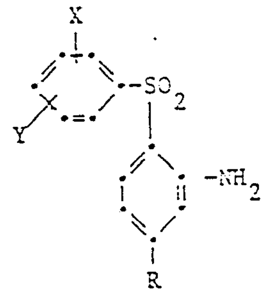

(4)

diazotiert und auf eine Kupplungskomponente der Formel

H—K (5)

kuppelt, wobei K, R, X und Y in den Formeln (4) und (5) die unter Formel (1) angegebenen Bedeutungen haben.

10. Verbindungen der Formel

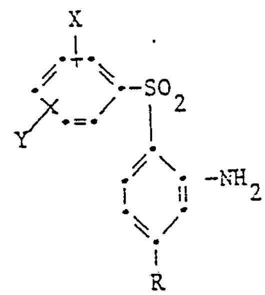

(4),

worin R eine

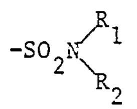 , 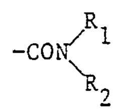 ,

—CO—$R_2$ oder —$SO_2$—O—$R_2$-Gruppe ist, wobei $R_1$ Wasserstoff oder $C_{1-4}$-Alkyl und $R_2$ einen gegebenenfalls substituierten Monosulfo-$C_{1-12}$-alkylrest, einen Rest der Formel

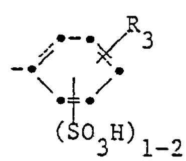

(2)

oder einen Rest der Formel

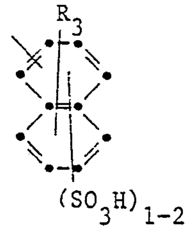

(3),

worin $R_3$ in den Formeln (2) und (3) Wasserstoff, $C_{1-4}$-Alkyl, $C_{1-4}$-Alkoxy, Halogen, $C_{2-6}$-Alkanoylamino oder Carboxy ist, und worin X und Y unabhängig voneinander Wasserstoff, $C_{1-4}$-Alkyl, $C_{1-4}$-Alkoxy, Halogen, $C_{2-6}$-Alkanoylamino, $C_{1-5}$-Alkylsulfonylamino oder einen gegebenenfalls substituierten Phenyl- oder Phenoxyrest bedeuten.

11. Verbindungen gemäss Anspruch 10, worin $R_2$ einen unsubstituierten Monosulfo-$C_{1-12}$-alkylrest oder einen Rest der Formel (2) oder (3) bedeutet und X und Y die in Anspruch 10 angegebenen Bedeutungen haben.

12. Verbindungen gemäss Anspruch 11, worin Y Wasserstoff, Chlor oder Methoxy ist und R und X die in Anspruch 11 angegebenen Bedeutungen haben.

13. Verbindungen gemäss Anspruch 12, worin X in p-Stellung zur —$SO_2$-Gruppe an den Phenylring gebunden ist und R und Y die in Anspruch 12 angegebenen Bedeutungen haben.

14. Verbindungen gemäss Anspruch 13, worin R eine

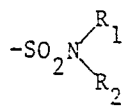 , 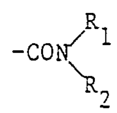 ,

—CO—$R_2$ oder —$SO_2$—O—$R_2$-Gruppe ist, wobei $R_1$ Wasserstoff oder Methyl und $R_2$ β-Sulfoäthyl, 2-, 3- oder 4-Sulfophenyl, wobei der Phenylring durch Methyl, Methoxy, Chlor, Acetylamino und Carboxy substituiert sein kann, 2,5-Disulfophenyl oder Monosulfo- oder Disulfonaphthyl ist, und worin X Wasserstoff, Methyl, Chlor, Aethoxy, Acetylamino oder Phenoxy und Y Wasserstoff, Chlor oder Methoxy ist.

15. Verbindungen gemäss Anspruch 14, worin R ein β-Sulfoäthylaminosulfonyl-, o-Sulfophenylaminosulfonyl-, p-Sulfophenylaminosulfonyl-, p-Sulfophenylaminocarbonyl- oder p-Methyl-m-sulfobenzoylrest, X Methyl oder Chlor und Y Wasserstoff ist.

16. Verfahren zur Herstellung von Verbindungen der Formel

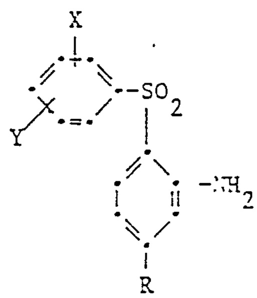

(4),

worin R, X und Y die in Anspruch 10 angegebenen Bedeutungen haben, dadurch gekennzeichnet, dass man eine Verbindung der Formel

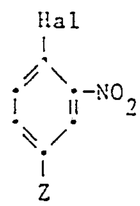

(6),

worin Z eine —$SO_2$—Hal oder —CO—Hal Gruppe ist und Hal Halogen bedeutet, mit einer Verbindung der Formel

$$[H(R_1)N,\ HO,\ H]—R_2 \qquad (7),$$

worin $R_1$ und $R_2$ die unter Formel (4) angegebenen Bedeutungen haben, zu einer Verbindung der Formel

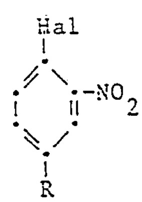

(8),

worin R die unter Formel (4) angegebene Bedeutung hat und Hal Halogen ist, umsetzt, und die Verbindung der Formel (8) mit einer Verbindung der Formel

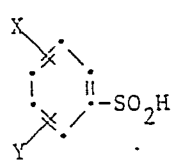

(9),

worin X und Y die unter Formel (4) angegebenen Bedeutungen haben, kondensiert und anschliessend die $NO_2$-Gruppe reduziert.

17. Verfahren zur Herstellung von Verbindungen der Formel

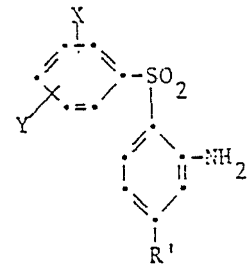

(10),

worin R′ eine

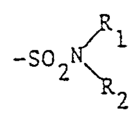 oder 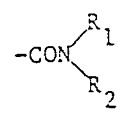

Gruppe ist, wobei $R_1$, $R_2$, X und Y die in Anspruch 10 angegebenen Bedeutungen haben, dadurch gekennzeichnet, dass man eine Verbindung der Formel

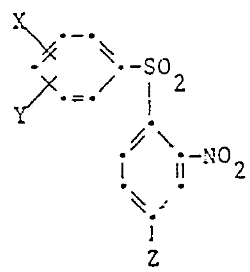

(11),

worin Z eine —$SO_2$—Hal oder —CO—Hal-Gruppe ist und Hal Halogen bedeutet, mit einem Amin der Formel

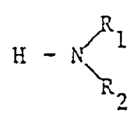

(12)

umsetzt und anschliessend die Nitrogruppe reduziert, wobei $R_1$, $R_2$, X und Y in den Formeln (11) und (12) die unter Formel (10) angegebenen Bedeutungen haben.

18. Verfahren zum Färben und Bedrucken unter Verwendung der Azofarbstoffe gemäss Anspruch 1.

19. Verfahren gemäss Anspruch 18 zum Färben von synthetischen und natürlichen Polyamidfasern.

20. Färbe- und Druckpräparate, die Azofarbstoffe gemäss den Ansprüchen 1 bis 6 enthalten.

**Claims**

1. A monoazo dye of the formula

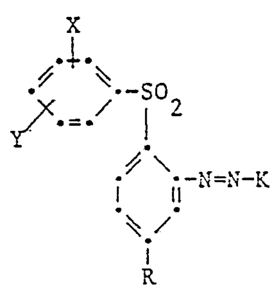

(1)

in which K is the radical of a coupling component of the benzene or naphthalene series or of the heterocyclic series, and R is a

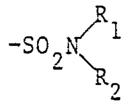 , 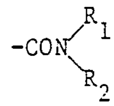 ,

—CO—$R_2$ or —$SO_2$—O—$R_2$ group in which $R_1$ is hydrogen or $C_{1-4}$alkyl and $R_2$ is a substituted or unsubstituted monosulfo-$C_{1-12}$alkyl radical, a radical of the formula

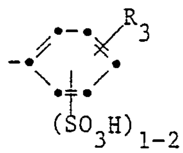 (2)

or a radical of the formula

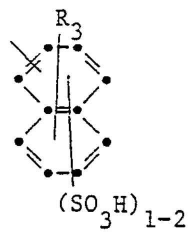 (3)

in which formulae (2) and (3) $R_3$ is hydrogen, $C_{1-4}$alkyl, $C_{1-4}$alkoxy, halogen, $C_{2-6}$alkanyolamino or carboxy, and in which formula (1) X and Y, independently of each other, are hydrogen, $C_{1-4}$alkyl, $C_{1-4}$-alkoxy, halogen, $C_{2-6}$alkanoylamino, $C_{1-5}$alkylsulfonylamino or a substituted or unsubstituted phenyl or phenoxy radical.

2. A monoazo dye according to claim 1, in which K is the radical of an aminobenzene, alkoxybenzene, aminonaphthalene, alkoxynaphthalene, naphthol, aminoaphthol, pyrazolone, aminopyrazole, pyridone, pyrimidine, indole, naphthimidazole, diphenylamine, pyrazolo[2,3-a]pyrimidine, tetrahydroquinoline or acetoacetamide, which radicals may be further substituted, and R, $R_1$, $R_2$, X and Y are as defined in claim 1.

3. A monoazo dye according to claim 1, in which $R_2$ is an unsubstituted monosulfo-$C_{1-12}$alkyl radical or a radical of the formula (2) or (3), and K, $R_1$, $R_3$, X and Y are as defined in claim 1.

4. A monoazo dye according to claim 3, in which Y is hydrogen, chlorine or methoxy, and K, R, $R_1$, $R_2$, $R_3$ and X are as defined in claim 3.

5. A monoazo dye according to claim 4, in which X is bonded to the phenyl ring in p-position relative to the —$SO_2$ group, and K, R, $R_1$, $R_2$, $R_3$ and Y are as defined in claim 4.

6. A monoazo dye according to claim 5, in which K is an N-$C_{1-4}$alkylaminobenzene or N,N-di-$c_{1-4}$-alkylaminobenzene radical which may be substituted in the benzene ring by methyl, chlorine, acetylamino and benzoylamino and in which the N-alkyl radicals may be substituted independently of each other by hydroxy, chlorine, cyano, phenyl or sulfophenyl ; a methoxybenzene radical ; a methoxynaphthalene radical ; an aminonaphthalene radical which may be substituted by sulfo ; a hydroxynaphthalene radical which may be substituted by sulfo ; an aminonaphthol radical which may be substituted in the naphthalene nucleus by sulfo and in which the amino group may be substituted by methyl, α,β-dibromopropionyl, α,β-dibromopropionylaminobenzoyl and 2,6-difluoro-5-chloropyrimidylaminobenzoyl ; a 1-phenyl-3-methylpyrazolone radical which may be substituted in the phenyl moiety by chlorine and methyl ; a 1-phenyl-3-methylaminopyrazole radical which may be substituted in the phenyl moiety by chlorine and sulfo ; a 1-ethyl-4-methyl-6-hydroxypyrid-2-one radical which may be substituted in the 3-position by carbamoyl ; a 2,4,6-triaminopyrimidine radical ; a 2-methylindole or 1-ethyl-2-methylindole radical ; a 1-chlorophenylamino-2-methylnaphthimidazole or 2-pentylnaphthimidazole radical which may be substituted in the naphthalene nucleus by hydroxy and sulfo ; an N-methyl- or N-ethyl-1,2,3,4-tetrahydroquinoline radical ; or a 2,5-dimethyl-7-aminopyrazolo[2,3-a]pyrimidine radical ; and R is a

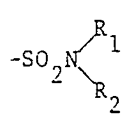 , 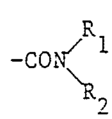 ,

—CO—$R_2$ or —$SO_2$—O—$R_2$ group in which $R_1$ is hydrogen or methyl ; and $R_2$ is β-sulfoethyl ; 2-, 3- or 4-sulfophenyl in which the phenyl ring may be substituted by methyl, methoxy, chlorine, acetylamino and carboxy ; 2,5-disulfophenyl, monosulfonaphthyl or disulfonaphthyl ; X is hydrogen, methyl, chlorine, ethoxy, acetylamino or phenoxy ; and Y is hydrogen, chlorine or methoxy.

7. A monoazo dye according to claim 6, in which K is an N,N-di-$C_{1-4}$alkylaminobenzene radical which may be substituted in the benzene nucleus by methyl, chlorine, acetylamino and benzoylamino and in which the N-alkyl moieties may be substituted independently of each other by hydroxy, chlorine, cyano, phenyl or sulfophenyl ; a 1-phenyl-3-methylaminopyrazole radical which may be substituted in the phenyl moiety by chlorine and sulfo ; or a 2-methylindole or 1-ethyl-2-methylindole radical ; and R, X and Y are as defined in claim 6.

8. A monoazo dye according to claim 7, in which K is an N,N-diethyl-3-methylaniline radical, an N-ethyl-N-β-hydroxyethyl-3-methylaniline radical, a 1-phenyl-3-methyl-5-aminopyrazole radical or a 2-methylindole radical, R is a β-sulfoethylaminosulfonyl, o-sulfophenylaminosulfonyl, p-sulfophenylaminosulfonyl, p-sulfophenylaminocarbonyl or p-methyl-m-sulfobenzoyl radical, X is methyl or chlorine, and Y is hydrogen.

9. A process for the preparation of a monoazo dye of the formula

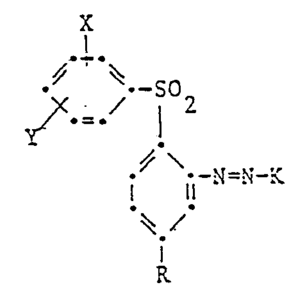

(1)

in which K, R, X and Y are as defined in claim 1, which process comprises diazotising a diazo component of the formula

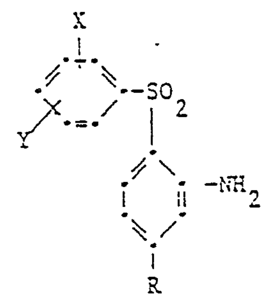

(4)

and coupling the product with a coupling component of the formula

H—K

in which formulae (4) and (5) K, R, X and Y are as defined for formula (1).

10. A compound of the formula

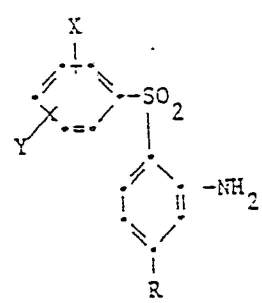

(4)

in which R is a

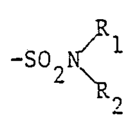 , 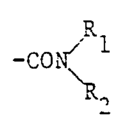 ,

—CO—$R_2$ or —$SO_2$—O—$R_2$ group in which $R_1$ is hydrogen or $C_{1-4}$alkyl, $R_2$ is a substituted or unsubstituted monosulfo-$C_{1-12}$alkyl radical, a radical of the formula

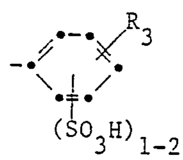
(2)

or a radical of the formula

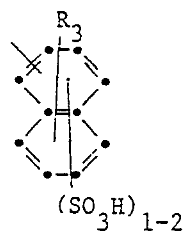
(3)

in which formulae (2) and (3) $R_3$ is hydrogen, $C_{1-4}$alkyl, $C_{1-4}$alkoxy, halogen, $C_{2-6}$alkanoylamino or carboxy, and in which formula (4) X and Y, independently of each other, are hydrogen, $C_{1-4}$alkyl, $C_{1-4}$alkoxy, halogen, $C_{2-6}$alkanoylamino, $C_{1-5}$alkylsulfonylamino or a substituted or unsubstituted phenyl or phenoxy radical.

11. A compound according to claim 10, in which $R_2$ is an unsubstituted monosulfo-$C_{1-12}$alkyl radical or a radical of the formula (2) or (3) and X and Y are as defined in claim 10.

12. A compound according to claim 11, in which Y is hydrogen, chlorine or methoxy, and R and X are as defined in claim 11.

13. A compound according to claim 12, in which X is bonded to the phenyl ring in p-position relative to the —$SO_2$ group, and R and Y are as defined in claim 12.

14. A compound according to claim 13, in which R is a

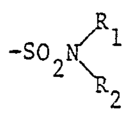 , 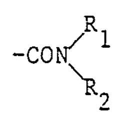 ,

—CO—$R_2$ or —$SO_2$—O—$R_2$ group in which $R_1$ is hydrogen or methyl ; $R_2$ is β-sulfoethyl ; 2-, 3- or 4-sulfophenyl in which the phenyl ring may be substituted by methyl, methoxy, chlorine, acetylamino and carboxy ; 2,5-disulfophenyl, monosulfonaphthyl or disulfonaphthyl ; and in which X is hydrogen, methyl, chlorine, ethoxy, acetylamino or phenoxy ; and Y is hydrogen, chlorine or methoxy.

15. A compound according to claim 14, in which R is a β-sulfoethylaminosulfonyl, o-sulfophenylaminosulfonyl, p-sulfophenylaminosulfonyl, p-sulfophenylaminocarbonyl or p-methyl-m-sulfobenzoyl radical, X is methyl or chlorine, and Y is hydrogen.

16. A process for the preparation of a compound of the formula :

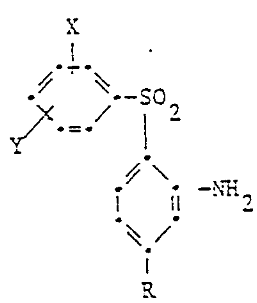
(4)

in which R, X and Y are as defined in claim 10, which process comprises reacting a compound of the formula

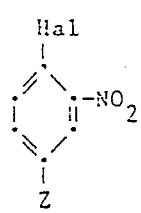
(6)

in which Z is a —$SO_2$—Hal or —CO—Hal group and Hal is halogen, with a compound of the formula

$$[H(R_1)N, HO, H]—R_2 \qquad (7)$$

in which $R_1$ and $R_2$ are as defined for formula (4), to give a compound of the formula

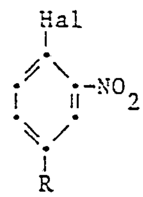

(8)

in which R is as defined for formula (4) and Hal is halogen, and condensing the compound of the formula (8) with a compound of the formula

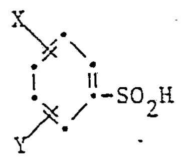

(9)

in which X and Y are as defined for formula (4), and then reducing the $NO_2$ group.

17. A process for the preparation of a compound of the formula

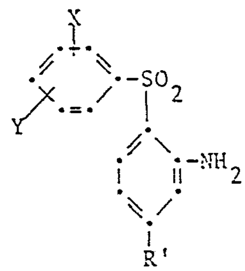

(10)

in which R′ is a

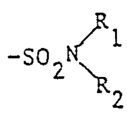 or 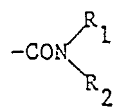

group, in which $R_1$, $R_2$, X and Y are as defined in claim 10, which process comprises reacting a compound of the formula

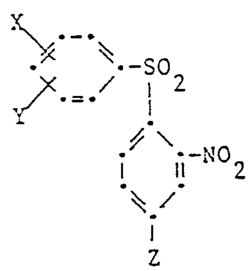

(11)

in which Z is a $—SO_2—$Hal or —CO—Hal group and Hal is halogen, with an amine of the formula

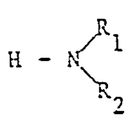

(12)

and then reducing the nitro group, in which formulae (11) and (12) $R_1$, $R_2$, X and Y are as defined for formula (10).

18. A process for dyeing or printing, which comprises the use of an azo dye according to claim 1.

19. A process according to claim 18, for dyeing synthetic and natural pclyamide fibres.

20. A dyeing or printing preparation which contains an azo dye according to any one of claims 1 to 6.

**Revendications**

1. Colorants mono-azoïques de formule

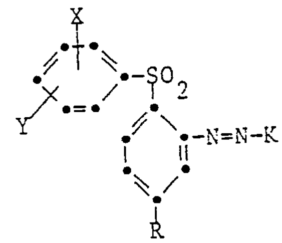

(1),

dans laquelle K est le reste d'un composant de copulation de la série du benzène ou de celle du naphtalène ou de la série hétérocyclique, et R est un groupement

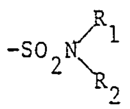 , 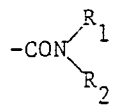 ,

—CO—$R_2$ ou —$SO_2$—O—$R_2$, $R_1$ représentant un atome d'hydrogène ou un radical alkyle en $C_{1-4}$ et $R_2$ représentant un radical monosulfo-alkyle en $C_{1-12}$ éventuellement substitué, un radical de formule

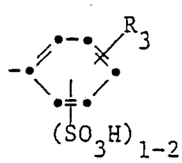

(2)

ou un radical de formule

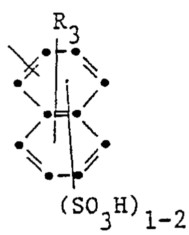

(3)

où $R_3$ est dans les formules (2) et (3) un atome d'hydrogène, un groupe alkyle en $C_{1-4}$, alcoxy en $C_{1-4}$, halogéno, alcanoyl($C_{2-6}$)-amino ou carboxyle, et dans laquelle X et Y représentent chacun, indépendamment l'un de l'autre, un atome d'hydrogène, un groupe alkyle en $C_{1-4}$, alcoxy en $C_{1-4}$, halogéno, alcanoyl($C_{2-6}$)-amino, alkyl($C_{1-5}$)-sulfonylamino ou un radical phényle ou phénoxy éventuellement substitué.

2. Colorants mono-azoïques selon la revendication.1, dans lesquels K est le reste d'un amino- ou alcoxybenzène, d'un amino- ou alcoxynaphtalène, d'un naphtol, d'un aminonaphtol, d'une pyrazolone, d'un aminopyrazole, d'une pyridone, d'une pyrimidine, d'un indole, d'un naphtimidazole, d'une diphénylamine, d'une pyrazolo(2,3-a)pyrimidine, d'une tétra-hydroquinoléine ou d'un acétylacétamide, les restes cités ci-dessus pouvant être substitués davantage, et R, $R_1$, $R_2$, X et Y ayant les significations données dans la revendication 1.

3. Colorants mono-azoïques selon la revendication 1, dans lesquels $R_2$ représente un reste monosulfo-alkyle en $C_{1-12}$ non substitué ou un reste de formule (2) ou de formule (3), et K, $R_1$, $R_3$, X et Y ont les significations données dans la revendication 1.

4. Colorants mono-azoïques selon la revendication 3, dans lesquels Y est un atome d'hydrogène, de chlore, ou un groupe méthoxy, et K, R, $R_1$, $R_2$, $R_3$ et X ont les significations données dans la revendication 3.

5. Colorants mono-azoïques selon la revendication 4, dans lesquels X est fixé au noyau phényle en position para par rapport au groupe —$SO_2$, et K, R, $R_1$, $R_2$, $R_3$ et Y ont les significations données dans la revendication 4.

6. Colorants mono-azoïques selon la revendication 5, dans lesquels K est un reste N-alkyl($C_{1-4}$)-amino- ou N,N-dialkyl($C_{1-4}$)-aminobenzène qui peut être substitué dans le noyau benzénique par un groupe méthyle, chloro, acétylamino et benzoylamino, et où les radicaux N-alkyle peuvent être substitués, indépendamment l'un de l'autre, par des groupes hydroxyle, chloro, cyano, phényle ou sulfophényle ; un reste méthoxybenzène ; un reste méthoxynaphtalène ; un reste aminophtalène qui peut être substitué par

un groupe sulfo ; un reste hydroxynaphtalène qui peut être substitué par un groupe sulfo ; un reste aminonaphtol qui peut être substitué dans le noyau naphtalène par un groupe sulfo, et dans lequel le radical amino peut être substitué par un groupe méthyle, α,β-dibromopropionyle, α,β-dibromopropionylaminobenzoyle et 2,6-difluoro-5-chloropyrimidylaminobenzoyle ; un reste 1-phényl-3-méthylpyrazolone qui peut être substitué dans le radical phényle par un atome de chlore et par un groupe méthyle ; un reste 1-phényl-3-méthyl-aminopyrazole qui peut être substitué dans le radical phényle par un atome de chlore et par un groupe sulfo ; un reste 1-éthyl-4-méthyl-6-hydroxy-pyridone-(2) qui peut être substitué en position 3 par un groupe carbamyle ; un reste 2,4,6-triaminopyrimidine ; un reste 2-méthylindole ou 1-éthyl-2-méthylindole ; un reste 1-chlorophénylamino-2-méthyl- ou 2-pentyl-naphtimidazole qui est substitué dans le noyau naphtalène par un groupe hydroxyle et par un groupe sulfo ; un reste N-méthyl- ou N-éthyl-1,2,3,4-tétra-hydroquinoléine ; ou un reste 2,5-diméthyl-7-amino-pyrazolo(2,3-a)pyrimidine ; et R est un groupement

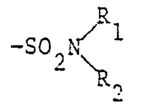 , 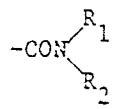 ,

$—CO—R_2$ ou $—SO_2—O—R_2$, $R_1$ étant un atome d'hydrogène ou un groupe méthyle, et R étant un groupe β-sulfo-éthyle, 2-, 3- ou 4-sulfophényle, le noyau phényle pouvant être substitué par un radical méthyle, méthoxy, chloro, acétylamino et carboxyle, un groupe 2,5-disulfophényle ou monosulfo- ou disulfonaphtyle, et dans lesquels X est un atome d'hydrogène, un groupe méthyle, chloro, éthoxy, acétylamino ou phénoxy, et Y est un atome d'hydrogène, de chlore, ou un groupe méthoxy.

7. Colorants mono-azoïques selon la revendication 6, dans lesquels K est un reste N,N-dialkyl($C_{1-4}$)-aminobenzène qui peut être substitué dans le noyau benzénique par un groupe méthyle, chloro, acétylamino et benzoylamino, et dans lequel les radicaux N-alkyle peuvent être, indépendamment l'un de l'autre, substitués par des groupes hydroxyle, chloro, cyano, phényle ou sulfophényle ; un reste 1-phényl-3-méthyl-aminopyrazole qui peut être substitué dans le radical phényle par un groupe chloro et par un groupe sulfo ; ou un reste 2-méthylindole ou 1-éthyl-2-méthylindole, et R, X et Y ont les significations données dans la revendication 6.

8. Colorants mono-azoïques selon la revendication 7, dans lesquels K est un reste N,N-diéthyl-3-méthylaniline, un reste N-éthyl-N-β-hydroxy-éthyl-3-méthylaniline, un reste 1-phényl-3-méthyl-5-aminopyrazole ou un reste 2-méthylindole ; R est un reste β-sulfo-éthylaminosulfonyle, o-sulfophénylaminosulfonyle, p-sulfophénylaminosulfonyle, p-sulfophénylaminocarbonyle ou p-méthyl-m-sulfobenzoyle ; X est un groupe méthyle ou chloro et Y est un atome d'hydrogène.

9. Procédé pour la préparation de colorants mono-azoïques de formule

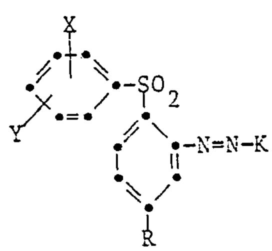 (1),

dans laquelle K, R, X et Y ont les significations données dans la revendication 1, caractérisé par le fait que l'on diazote un composant diazoïque de formule

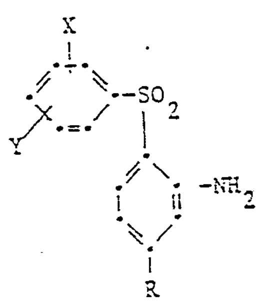 (4)

et qu'on le fait copuler avec un composant de copulation de formule

H—K (5),

K, R, X et Y ayant dans les formules (4) et (5) les significations données pour la formule (1).

10. Composés de formule

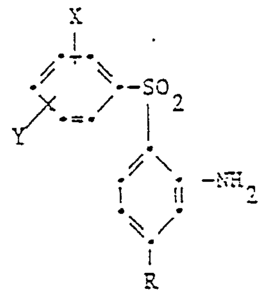

(4),

dans laquelle R est un groupement

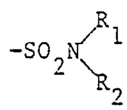 , 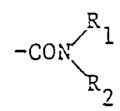 ,

—CO—$R_2$ ou —$SO_2$—O—$R_2$, R étant un atome d'hydrogène ou un groupe alkyle en $C_{1\text{-}4}$ et $R_2$ étant un reste monosulfo-alkyle en $C_{1\text{-}12}$ éventuellement substitué, un reste de formule

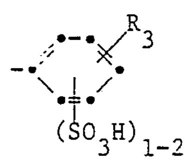

(2)

ou un reste de formule

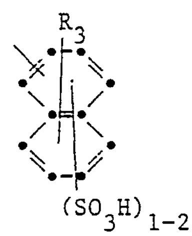

(3),

où $R_3$ est dans les formules (2) et (3) un atome d'hydrogène, un groupe alkyle en $C_{1\text{-}4}$, alcoxy en $C_{1\text{-}4}$, halogéno, alcanoyl($C_{2\text{-}6}$)-amino ou carboxyle, et dans laquelle X et Y représentent chacun, indépendamment l'un de l'autre, un atome d'hydrogène, un groupe alkyle en $C_{1\text{-}4}$, alcoxy en $C_{1\text{-}4}$, halogéno, alcanoyl($C_{2\text{-}6}$)-amino, alkyl($C_{1\text{-}5}$)-sulfonylamino ou un reste phényle ou phénoxy éventuellement substitué.

11. Composés selon la revendication 10, dans lesquels $R_2$ représente un reste monosulfo-alkyle en $C_{1\text{-}12}$ non substitué ou un reste de formule (2) ou de formule (3), et X et Y ont les significations données dans la revendication 10.

12. Composés selon la revendication 11, dans lesquels Y est un atome d'hydrogène, de chlore, ou un groupe méthoxy et R et X ont les significations données dans la revendication 11.

13. Composés selon la revendication 12, dans lesquels X est fixé au noyau phényle en position para par rapport au groupe —$SO_2$ et R et Y ont les significations données dans la revendication 12.

14. Composés selon la revendication 13, dans lesquels R est un groupement

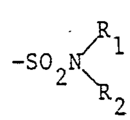 , 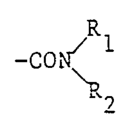 ,

—CO—$R_2$ ou —$SO_2$—O—$R_2$, $R_1$ étant un atome d'hydrogène ou un groupe méthyle, et $R_2$ étant un groupe β-sulfo-éthyle, 2-, 3- ou 4-sulfophényle, le noyau phényle pouvant être substitué par un radical méthyle, méthoxy, chloro, acétylamino et carboxyle, un groupe 2,5-disulfophényle ou monosulfo- ou disulfonaphtyle, et dans lesquels X est un atome d'hydrogène, un groupe méthyle, chloro, éthoxy, acétylamino ou phénoxy, et Y est un atome d'hydrogène, de chlore, ou un groupe méthoxy.

15. Composés selon la revendication 14, dans lesquels R est un reste β-sulfo-éthylaminosulfonyle, o-sulfophénylaminosufonyle, p-sulfophénylaminosulfonyle, p-sulfophénylaminocarbonyle ou p-méthyl-m-sulfobenzoyle, X est un groupe méthyle ou chloro et Y est un atome d'hydrogène.

16. Procédé pour la préparation de composés de formule

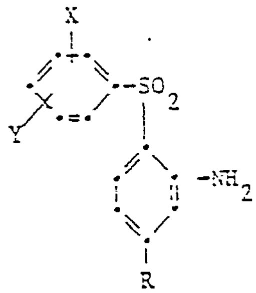

(4),

dans laquelle R, X et Y ont les significations données dans la revendication 10, caractérisé par le fait que l'on fait réagir un composé de formule

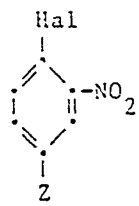

(6),

dans laquelle Z est un groupe $—SO_2—Hal$ ou $—CO—Hal$ et Hal représente un halogène, avec un composé de formule

$$[H(R_1)N,\ HO,\ H]—R_2 \qquad (7),$$

dans laquelle $R_1$ et $R_2$ ont les significations données pour la formule (4), pour aboutir à un composé de formule

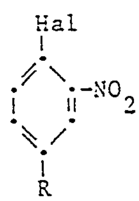

(8),

dans laquelle R a la signification donnée pour la formule (4) et Hal est un halogène, et que l'on condense le composé de formule (8) avec un composé de formule

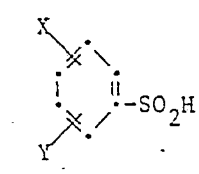

(9),

dans laquelle X et Y ont les significations données pour la formule (4), et que l'on réduit ensuite le groupe $NO_2$.

17. Procédé pour la préparation de composés de formule

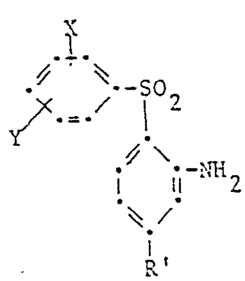

(10),

dans laquelle R' est un groupe

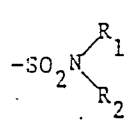

ou

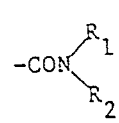

$R_1$, $R_2$, X et Y ayant les significations données dans la revendication 10, caractérisé par le fait que l'on fait réagir un composé de formule

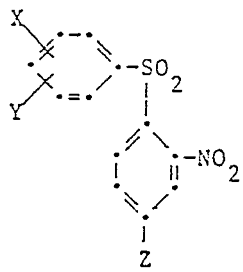

(11),

dans laquelle Z est un groupe —$SO_2$—Hal ou —CO—Hal et Hal représente un halogène, avec une amine de formule

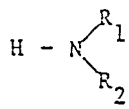

(12)

et que l'on réduit ensuite le groupe nitro, $R_1$, $R_2$, X et Y ayant dans les formules (11) et (12) les significations données pour la formule (10).

18. Procédé pour la teinture et l'impression par utilisation des colorants azoïques selon la revendication 1.

19. Procédé selon la revendication 18 pour la teinture de fibres de polyamide synthétiques et naturelles.

20. Préparations tinctoriales et pour l'impression, qui contiennent des colorants azoïques selon les revendications 1 à 6.